# EUROPEAN PATENT APPLICATION

(11) **EP 4 375 670 A1**
(43) Date of publication of application: **29.05.2024**
(21) Application number: 22842474.3
(22) Date of filing: 14.07.2022
(51) Int. Cl.: G01N 33/68

(54) **EPITOPE OF REGULATORY T CELL SURFACE ANTIGEN, AND ANTIBODY SPECIFICALLY BINDING THERETO**

(30) Priority: 16.07.2021 KR 20210093657
(71) Applicant: Good T Cells, Inc., Seoul 03929 (KR)
(72) Inventor: KIM, Jung Ho, Seoul 04136 (KR); KIM, Beom Seok, Seoul 03929 (KR)
(74) Representative: Latscha Schöllhorn Partner AG
(86) International application number: PCT/KR2022/010262
(87) International publication number: WO 2023/287212

(57) **Abstract**

The present invention relates to an epitope of Lrig1 (leucine-rich and immunoglobulin-like domains protein 1) protein, which is an antigen present on the surface of regulatory T cells, and to an antibody or an antigen-binding fragment that specifically binds thereto.

## Description

### Technical Field

The present invention relates to an epitope of Lrig-1 (leucine-rich and immunoglobulin-like domains 1) protein, which is an antigen present on the surfaces of regulatory T cells, and an antibody or antigen-binding fragment that binds specifically thereto.

### Background Art

One of the most important traits in all normal individuals is their ability to recognize and eliminate non-self antigens while not reacting harmfully to self-antigenic substances that make up the self. As such, the non-response of a living body to self-antigens is called immunologic unresponsiveness or tolerance. Self-tolerance occurs either by elimination of lymphocytes that may have specific receptors for self-antigens or by inactivation of self-reactive lymphocytes after contact with self-antigens. Abnormalities in the induction or maintenance of self-tolerance lead to immune responses against self-antigens, which may result in disorders called autoimmune diseases.

For the treatment of the autoimmune disease, the concept of suppressor T cells with the possibility of presence of T cells capable of controlling and suppressing the effector function of conventional T cells was first introduced and proposed by Gershon in the early 1970s. Since then, research has been conducted to elucidate the biological properties and functions of regulatory T cells in many fields of immunology.

Accordingly, the regulatory T cells (Treg) play an important role in naturally preventing the occurrence of excessive inflammation and immune responses, but it has been reported that, when autoimmune diseases and chronic inflammatory diseases occur, the function and number of regulatory T cells remarkably decrease. Thus, for patients with immune diseases and inflammatory diseases, it is important that regulatory T cells are generated at normal levels, which can be one of methods for treatment of these diseases.

Until now, studies on genes and proteins present specifically in regulatory T cells have been conducted, suggesting that substances such as CD25, CTLA4, CD62L, CD38, CD103, GITR, and CD45RB may correspond to marker substances. However, genes or proteins that can target regulatory T cells alone have not yet been reported.

Meanwhile, there are three hypervariable regions, called complementarity determining regions (hereinafter referred to as "CDRs"), and four framework regions. The CDRs mainly serve to bind to an antigenic determinant (epitope) of an antigen. The CDRs of each chain are typically referred to as CDR1, CDR2, and CDR3, numbered sequentially starting from the N-terminus, and are also identified by the chain in which the particular CDR is located.

### DISCLOSURE

### Technical Problem

An object of the present invention is to provide an epitope of Lrig-1 (leucine-rich and immunoglobulin-like domains 1) protein present on the surface of a regulatory T cell (Treg cell).

Another object of the present invention is to provide an antibody or antigen-binding fragment capable of binding specifically to the epitope.

Still another object of the present invention is to provide a nucleic acid molecule encoding the epitope of the present invention; an expression vector into which the nucleic acid molecule has been inserted; and a host cell line transfected with the expression vector.

Yet another object of the present invention is to provide a pharmaceutical composition for preventing or treating various diseases comprising, as an active ingredient, the antibody or antigen-binding fragment capable of specifically binding to the epitope.

Still yet another object of the present invention is to provide an antibody-drug conjugate (ADC) in which a drug for preventing or treating various diseases is conjugated to the antibody according to the present invention.

A further object of the present invention is to provide a pharmaceutical composition for preventing or treating various diseases, such as immune-related disease, neurodegenerative disease, or neuroinflammatory disease, the pharmaceutical composition comprising the antibody-drug conjugate as an active ingredient.

Another further object of the present invention is to provide a method of preventing or treating various diseases using the antibody or antigen-binding fragment capable of binding specifically to the epitope, and the antibody-drug conjugate.

However, objects to be achieved by the present invention are not limited to the objects mentioned above, and other objects not mentioned above will be clearly understood by those of ordinary skill in the art from the following description.

### Technical Solution

The present invention is directed to an epitope of Lrig-1 (leucine-rich and immunoglobulin-like domains 1) protein or an antibody or antigen-binding fragment that binds specifically to the epitope.

In the present invention, the "epitope" refers to a sequence of amino acids in an antigen wherein the amino acids (or a subset thereof) in the sequence are specifically recognized by an antibody or binding fragment, for example an antibody or binding fragment described herein. An epitope may comprise one or more antigenic determinants. For example, an antibody generated against an isolated peptide corresponding to a conformational epitope recognizes part or all of said epitope sequence.

In the present invention, the "Lrig-1 protein" is a transmembrane protein present on the surfaces of regulatory T cells, and is composed of leucine-rich repeats (LRRs) and three immunoglobulin-like domains on the extracellular or lumen side, a cell transmembrane sequence, and a cytoplasmic tail region. The LRIG gene family includes LRIG1, LRIG2, and LRIG3, and the amino acids constituting each member of the family are highly conserved. The LRIG1 gene is highly expressed in normal skin, and can be expressed in basal and hair follicle cells to regulate proliferation of epithelial stem cells. Thus, the LRIG1 gene plays an important role in maintaining homeostasis of the epidermis, and its absence may lead to psoriasis or skin cancer. It has been reported that a deletion of chromosome region 3p14.3 in which LRIG1 is located is likely to develop into cancer cells. In fact, it was found that expression of LRIG1 greatly decreased in renal cell carcinoma and cutaneous squamous cell carcinoma. In recent years, it has also been found that the Lrig-1 protein is expressed in only about 20 to 30% of cancers. Meanwhile, for the purpose of the present invention, the Lrig-1 protein may be a protein derived from mammals or mice, without being limited thereto.

As an example of the present invention, the Lrig-1 protein may be a Lrig-1 protein derived from mammals, including primates such as humans and monkeys, rodents such as mice and rats, or the like.

As an example of the present invention, the Lrig-1 protein may be a human-derived Lrig-1 protein represented by SEQ ID NO: 1, which may be encoded by the nucleic acid sequence represented by SEQ ID NO: 2, without being limited thereto.

As another example of the present invention, the Lrig-1 protein may be a mouse-derived Lrig-1 protein represented by SEQ ID NO: 3, which may be encoded by the nucleic acid sequence represented by SEQ ID NO: 4, without being limited thereto.

As another example of the present invention, the Lrig-1 protein may be an extracellular domain of the Lrig-1 protein, without being limited thereto.

The Lrig-1 extracellular domain in the present invention may be an extracellular domain of Lrig-1 protein derived from mammals including primates such as humans and monkeys, rodents such as mice and rats, or the like. For the purpose of the present invention, the extracellular protein of the Lrig-1 protein may be an extracellular domain of Lrig-1 protein derived from a human or mouse, without being limited thereto.

As an example of the present invention, the extracellular domain of the Lrig-1 protein may be represented by SEQ ID NO: 5, which corresponds to amino acid positions 35 to 794 in the amino acid sequence of the human-derived Lrig-1 protein, without being limited thereto.

As another example of the present invention, the extracellular domain of the Lrig-1 protein may be represented by SEQ ID NO: 6, which corresponds to amino acid positions 35 to 794 in the amino acid sequence of the mouse-derived Lrig-1 protein, without being limited thereto.

Hereinafter, the present invention will be described in more detail.

In accordance with one embodiment of the present invention, there is provided an epitope of the Lrig-1 protein, the epitope comprising at least one polypeptide selected from the group consisting of polypeptides consisting of the amino acid sequence represented by SEQ ID NOs: 35 to 45.

As an example of the present invention, the epitope of the Lirg-1 protein may be an epitope comprising at least one polypeptide selected from the group consisting of polypeptides consisting of the amino acid sequence represented by SEQ ID NOs: 35 to 40, without being limited thereto.

As another example of the present invention, the epitope of the Lirg-1 protein may be an epitope comprising at least one polypeptide selected from the group consisting of polypeptides consisting of the amino acid sequence represented by SEQ ID NOs: 41 to 45, without being limited thereto.

According to one embodiment of the present invention, the epitope of the Lrig-1 protein may be a region corresponding to the amino acid sequence at positions 62 to 101, or 88 to 92, or 90 to 101, or 111 to 134, or 452 to 476, or 472 to 480, or 542 to 553 of the Lrig-1 protein.

According to one embodiment of the present invention, the epitope of the Lrig-1 protein may be a region corresponding to the amino acid sequence at positions 95 to 101, or 472 to 476 of the Lrig-1 protein.

The epitope of the present invention may be a conformational epitope.

The "conformational epitope" of the present invention consists of a discontinuous amino acid sequence, unlike a one-dimensional linear epitope consisting of a continuous sequence. This conformational epitope reacts with the three-dimensional structure of the antigen-binding site of an antibody.

Another embodiment of the present invention provides a nucleic acid molecule encoding the epitope provided in the present invention.

Nucleic acid molecules of the present invention include all nucleic acid molecules having polynucleotide sequences which are translated into the amino acid sequences of the polypeptides provided in the present invention, as known to those skilled in the art. Therefore, various polynucleotide sequences with open reading frames (ORFs) may be produced, which are also all included in the nucleic acid molecules of the present invention.

Another embodiment of the present invention provides an expression vector into which the isolated nucleic acid molecule provided in the present invention has been inserted.

In the present invention, the "vector" is any nucleic acid molecule capable of transporting another nucleic acid linked thereto. One type of vector is a "plasmid," which refers to a circular double-stranded DNA to which an additional DNA segment may be ligated. Another type of vector is a phage vector. Yet another type of vector is a viral vector, where an additional DNA segment can be ligated into the viral genome. Certain vectors are capable of autonomous replication in a host cell into which they have been introduced (for example, bacterial vectors having a bacterial origin of replication are episomal mammalian vectors). Other vectors (e.g., non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, the terms "plasmid" and "vector" may be used interchangeably as the plasmid is the most commonly used form of vector.

Specific examples of the expression vector in the present invention may be selected from the group consisting of commercially widely used pCDNA vectors, F, R1, RP1, Col, pBR322, ToL, Ti vectors; cosmids; phages such as lambda, lambdoid, M13, Mu, p1 P22, Qµµ, T-even, T2, T3, T7; and plant viruses, without being limited thereto. Any expression vector known as expression vectors to those skilled in the art may be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation, without being limited thereto, and those skilled in the art may adopt and use an introduction method appropriate for the expression vector and host cell that are used. The vector may preferably contain at least one selection marker, without being limited thereto, and selection can be made using a vector that contains no selection marker, depending on whether or not a product is produced. The selection marker is chosen depending on the target host cell, which is done using methods already known to those skilled in the art, and thus the present invention has no limitation thereon.

In order to facilitate purification of the protein encoded by the nucleic acid molecule of the present invention, a tag sequence may be inserted into and fused to the expression vector. Examples of the tag include, but are not limited to, hexa-histidine tag, hemagglutinin tag, myc tag, or flag tag, and any tag known to those skilled in the art which facilitates purification may be used in the present invention.

Another embodiment of the present invention provides a host cell line transformed with the expression vector provided in the present invention.

In the present invention, the term "host cell" includes individual cells or cell cultures which may be or have been recipients of the vector(s) for incorporation of a polypeptide insert. The host cells include progeny of a single host cell, and the progeny may not necessarily be completely identical (in morphology or in genomic DNA complement) to the original parent cell due to natural, accidental, or intentional mutation. The host cells include cells transfected *in vivo* with the polynucleotide(s) of the present invention.

In the present invention, the host cells may include cells of mammalian, plant, insect, fungal, or cellular origin. For example, the host cells may be bacterial cells such as *E. coli, Streptomyces*, *Salmonella typhimurium*; fungal cells such as yeast cells and *Pichia pastoris*; insect cells such as Drosophila and *Spodoptera* Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells, without being limited thereto. In addition, any cell type known to those skilled in the art which may be used as a host cell line may be used.

The transformation method of the present invention is any method of introducing a desired vector into the host cell, and may include any known method capable of introducing the vector into the host cell. For example, a method using CaCl₂, electroporation, microinjection, calcium phosphate precipitation, electroporation, liposome-mediated transfection, DEAE-dextran treatment, gene bombardment, and transfection using virus, may be used, without being limited thereto.

Another embodiment of the present invention provides a binding molecule that binds specifically to an epitope of the present invention.

The "binding molecule" of the present invention may be either an antibody or an antigen-binding fragment, without being limited thereto.

In the present invention, the antibody is a full-length antibody or a part of the antibody, has the ability to bind to the Lrig-1 protein, and includes any antibody fragment that binds to the Lrig-1 antigen determining region in a manner competitive with the binding molecule of the present invention.

In the present invention, the term "antibody" refers to a protein molecule which serves as a receptor that specifically recognizes an antigen, including an immunoglobulin molecule that is immunologically reactive with a particular antigen. For the purpose of the present invention, the antigen may be Lrig-1 protein present on the surfaces of regulatory T cells. Preferably, the antibody may be one that specifically recognizes the leucine-rich region or immunoglobulin-like domain of the Lrig-1 protein, without being limited thereto.

In the present invention, the "immunoglobulin" has a heavy chain and a light chain, and each of the heavy chain and the light chain comprises a constant region and a variable region. The variable region of each of the light chain and the heavy chain contains three hypervariable regions, called complementarity determining regions (hereinafter referred to as "CDRs"), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the antibody or antigen-binding fragment thereof provided in the present invention is a chimeric antibody or fragment comprising heavy chain and light chain CDRs selected from the CDRs provided in the present invention, or conservative variants of the CDRs provided in the present invention.

For the purpose of the present invention, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, but random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably, the degree of similarity, preferably identity between a given amino acid sequence and an amino acid sequence which is a variant of said given amino acid sequence will be at least about 60%, 65%, 70%, 80%, 81%, 82%, 83%, 84%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with tools known in the art, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm, or by means of a similarity search method, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wisconsin, USA.

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit, according to the present invention, at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by those skilled in the art, for example, by recombinant DNA manipulation. The manipulation of DNA sequences for preparing peptides or proteins having substitutions, additions, insertions or deletions, is well known. Furthermore, the peptides and amino acid variants described herein may be readily prepared with the aid of known peptide synthesis techniques, for example, by solid phase synthesis and similar methods.

In the present invention, the antibody or antigen binding fragment thereof provided in the present invention is a humanized antibody or fragment comprising heavy and light chain CDRs selected from the CDRs provided in the present invention, or conservative variants of the CDRs provided in the present invention.

In the present invention, the antibody or antigen binding fragment thereof provided in the present invention comprises a light chain and/or a heavy chain comprising a sequence provided in the present invention, or a conservative variant thereof. In one embodiment, the conservative variants have at least 80%, at least 85%, at least 90%, at least 95%, at least 96%, at least 97%, at least 98% or at least 99% homology to the reference sequence provided in the present invention. In one embodiment, the conservative variants comprise 1, 2, 3, 4, 5, 6, 7, 8, 9, 10, or more amino acid substitutions, insertions, or deletions.

In the present invention, the antibodies provided in the present invention, which bind specifically to the Lrig-1 protein comprise the sequences provided herein or conservative variants thereof. "Conservative variants," as used herein, include conservative amino acid substitutions, insertions, or deletions. A person skilled in the art will recognize that a conservative amino acid substitution is a substitution of one amino acid with another amino acid that has similar structural or chemical properties, such as a similar side chain, and a conservative amino acid substitution results in a sequence that retains the biological activity of the reference sequence. Exemplary conservative substitutions are well known in the art.

In the present invention, the "full-length antibody" has a structure having two full-length light chains and two full-length heavy chains, wherein each of the light chains is linked to the heavy chain via a disulfide bond. Examples of the full-length antibody include IgA, IgD, IgE, IgM, and IgG. Subtypes of the IgG include IgG1, IgG2, IgG3, and IgG4.

In addition, in the present invention, the "antigen-binding fragment" refers to a fragment having an antigen-binding function. Examples of the antigen-binding fragment include: (1) a Fab fragment consisting of a light chain variable region (VL), a heavy chain variable region (VH), a light chain constant region (CL), and a heavy chain constant region 1 (CH1); (2) a Fd fragment consisting of VH and CH1 domains; (3) a Fv fragment consisting of VL and VH domains of a single antibody; (4) a dAb fragment consisting of a VH domain; (5) an isolated CDR region; (6) a F(ab')₂ fragment which is a bivalent fragment including two linked Fab fragments; (7) a single-chain Fv molecule (scFv) in which a VH domain and a VL domain are linked together by a peptide linker that allows the two domains to associate to form an antigen-binding site; (8) a bispecific single-chain Fv dimer; and (9) a diabody which is a multivalent or multispecific fragment constructed by gene fusion. The antigen-binding fragment may be obtained as a Fab or F(ab')₂ fragment when a proteolytic enzyme, for example, papain or pepsin is used, and the antigen-binding fragment may be produced through a genetic recombination technique.

In addition, in the present invention, the antibody or a fragment thereof may be, without limitation, a monoclonal antibody, a polyclonal antibody, a chimeric antibody, a humanized antibody, a human antibody, a bivalent-bispecific molecule, a minibody, a domain antibody, a bispecific antibody, an antibody mimetic, a unibody, a diabody, a triabody, a tetrabody, or a fragment thereof, without being limited thereto.

In the present invention, the "chimeric antibody" is an antibody which is obtained by recombination of a variable region of a mouse antibody and a constant region of a human antibody, and has a greatly improved immune response compared to the mouse antibody.

In addition, in the present invention, the "humanized antibody" refers to an antibody obtained by modifying the protein sequence of an antibody of non-human species origin so that the protein sequence is similar to an antibody variant naturally produced in humans. For example, the humanized antibody may be produced by recombining mouse-derived CDRs with a human antibody-derived FR to obtain a humanized variable region, and recombining the humanized variable region with a constant region of a desired human antibody.

In the present invention, the binding molecule may be provided as a bispecific antibody or bispecific antigen-binding fragment which is capable of binding to Lrig-1 protein and also binding to another protein.

In the present invention, the bispecific antibody and the bispecific antigen-binding fragment may comprise the binding molecule according to the present invention. As an example of the present invention, the bispecific antibody and the bispecific antigen-binding fragment comprise an antigen-binding domain capable of binding to Lrig-1 protein, wherein the antigen-binding domain capable of binding to Lrig-1 protein may comprise or consist of the binding molecule according to the present invention.

The bispecific antibody and bispecific antigen-binding fragment provided in the present invention comprise an antigen-binding domain, which is a binding molecule capable of binding to Lrig-1 protein according to the present invention, and an antigen-binding domain capable of binding to another target protein. Here, the antigen-binding domain capable of binding to another target protein may be an antigen-binding domain capable of binding to a protein other than Lrig-1 protein, for example, but not limited to, PD-1 or a cell surface receptor. However, the antigen-binding domain is not limited thereto.

The bispecific antibody and the bispecific antigen-binding fragment according to the present invention may be provided in any suitable format, for example, that described in the literature, which is incorporated herein by reference in its entirety. For example, the bispecific antibody or the bispecific antigen-binding fragment may be a bispecific antibody conjugate (for example, IgG2, F(ab')2, or CovX-body), a bispecific IgG or IgG-like molecule (for example, IgG, scFv4-Ig, IgG-scFv, scFv-IgG, DVD-Ig, IgG-sVD, sVD-IgG, or 2 in 1-IgG, mAb2, or Tandemab common LC), an asymmetric bispecific IgG or IgG-like molecule (for example, kih IgG, kih IgG common LC, CrossMab, kih IgG-scFab, mAb-Fv, charge pair, or SEED-body), a small bispecific antibody molecule (for example, diabody (Db), dsDb, DART, scDb, tandAb, tandem scFv (taFv), tandem dAb/VHH, triple body, triple head, Fab-scFv, or F(ab')2-scFv2), a bispecific Fc and CH3 fusion protein (for example, taFv-Fc, di-diabody, scDb-CH3, scFv-Fc-scFv, HCAb-VHH, scFv-kih-Fc, or scFv-kih-CH3), or a bispecific fusion protein (for example, scFv2-albumin, scDb-albumin, taFv-toxin, DNL-Fab3, DNL-Fab4-IgG, DNL-Fab4-IgG-cytokine 2). The bispecific antibody and bispecific antigen-binding fragment according to the present invention may be designed and produced by those skilled in the art.

In the present invention, a method for producing the bispecific antibody comprises chemical crosslinking of an antibody or antibody fragment with a reducing disulfide or non-reducing thioether bond. For example, N-succinimidyl-3-(-2-pyridyldithio)-propionate (SPDP) may be used, for example, for chemically crosslinking an Fab fragment through an SH-group at the hinge region, to generate a disulfide-linked bispecific F(ab)2 heterodimer.

In addition, an alternative method for producing the bispecific antibody according to the present invention comprises fusing an antibody-producing hybridoma with, for example, polyethylene glycol, to produce quadroma cells capable of secreting bispecific antibodies.

The bispecific antibody and bispecific antigen-binding fragment according to the invention may also be, for example, recombinantly produced by expression from a nucleic acid construct that encodes a polypeptide for an antigen-binding molecule.

For example, a DNA construct that contains a sequence encoding light and heavy chain variable domains for two antigen-binding domains (that is, light and heavy chain variable domains for an antigen-binding domain capable of binding to PD-1, and light and heavy chain variable domains for an antigen-binding domain capable of binding to another target protein), and a sequence encoding a suitable linker or dimerization domain between the antigen-binding domains may be prepared by molecular cloning techniques. Subsequently, a recombinant bispecific antibody may be produced by expression of the construct (for example, *in vitro*) in a suitable host cell (for example, a mammalian host cell), and then the expressed recombinant bispecific antibody may be optionally purified.

Antibodies may be produced by an affinity maturation process in which a modified antibody with improved affinity for an antigen as compared with an unmodified parent antibody is produced. An affinity-matured antibody may be produced by a procedure known in the art.

In addition, the binding molecule provided in the present invention may include a variant of the amino acid sequence as long as the variant can specifically bind to Lrig-1 protein. For example, in order to improve binding affinity and/or other biological properties of an antibody, modifications may be made to an amino acid sequence of the antibody. Such modifications include, for example, deletions, insertions, and/or substitutions of amino acid sequence residues of the antibody.

Such amino acid variations are made based on relative similarity of amino acid side chain substituents such as hydrophobicity, hydrophilicity, charge, and size. According to analysis on sizes, shapes, and types of amino acid side chain substituents, it can be seen that arginine, lysine, and histidine are all positively charged residues; alanine, glycine, and serine have similar sizes; and phenylalanine, tryptophan, and tyrosine have similar shapes. Thus, based on these considerations, it can be said that arginine, lysine, and histidine; alanine, glycine, and serine; and phenylalanine, tryptophan, and tyrosine are biologically functional equivalents.

To introduce variations, the hydropathic index of amino acids may be considered. Each amino acid has been assigned a hydropathic index on the basis of their hydrophobicity and charge characteristics, which are as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). the hydropathic amino acid index is very important in conferring interactive biological function on a protein. It is known that certain amino acids may be substituted for other amino acids having a similar hydropathic index and still retain a similar biological activity. To introduce variations based on the hydropathic index, substitutions are made between amino acids that exhibit a hydropathic index difference of preferably within ±2, more preferably ±1, even more preferably ±0.5.

Meanwhile, it is also well known that substitution between amino acids having similar hydrophilicity values results in proteins having equivalent biological activity. As disclosed in U.S. Pat. No. 4,554,101, the following hydrophilicity values have been assigned to amino acid residues: arginine (+3.0); lysine (+3.0); aspartate (+3.0+1); glutamate (+3.0+1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). To introduce variations based on the hydrophilicity value, substitutions may be made between amino acids that exhibit a hydrophilicity value difference of preferably within ±2, more preferably ±1, even more preferably ±0.5.

Amino acid exchanges in proteins, which do not generally alter the activity of molecules, are known in the art. The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, and Gln/Glu.

Considering the above-described variations having biologically equivalent activity, it is interpreted that the binding molecules of the present invention also include a sequence showing substantial identity with the sequence set forth in the Sequence Listing.

In the present invention, the term "substantial identity" means a sequence showing at least 61% homology, more preferably 70% homology, even more preferably 80% homology, most preferably 90% homology, as determined by aligning the sequence of the present invention with any other sequence to correspond to each other as much as possible and analyzing the aligned sequence using an algorithm commonly used in the art. Alignment methods for sequence comparison are known in the art. Various methods and algorithms for alignment are accessible from NCBI Basic Local Alignment Search Tool (BLAST), NCBI (National Center for Biological Information), etc., and may be used in conjunction with sequencing programs, such as blastp, blasm, blastx, tblastn and tblastx, on the Internet. BLAST is available from http://www.ncbi.nlm.nih.gov/BLAST/. Sequence homology comparison methods using this program can be identified online (http://www.ncbi.nlm.nih.gov/BLAST/blast_help.html).

In the present invention, although the binding molecule, preferably the antibody, may be produced by a conventional method for producing an antibody, it may be produced by affinity maturation.

In the present invention, the "affinity maturation" refers to a process in which antibodies having increased affinity for an antigen are produced by activated B cells in the course of an immune response. For the purpose of the present invention, the affinity maturation allows antibodies or antibody fragments to be produced due to affinity maturation based on the principles of mutation and selection, in the same process as that occurring in nature.

In addition, in the present invention, the "monoclonal antibody" refers to an antibody molecule of a single molecular composition which is obtained from substantially the same antibody population, and exhibits single binding specificity and affinity for a particular epitope.

In the present invention, the "binding" or "specific binding" refers to the affinity of the antibody or antibody composition herein for an antigen. In antigen-antibody binding, the "specific binding" is distinguishable from non-specific background binding, typically in a case where a dissociation constant (Kd) is less than 1×10⁻⁵ M, less than 1×10⁻⁶ M, or less than 1×10⁻⁷ M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least part of antigen-binding capacity of a monomer. Such a multimer also includes a homomultimer or a heteromultimer. Antibody multimers contain a large number of antigen-binding sites, and thus have superior antigen-binding capacity as compared with monomers. Antibody multimers are also easily used to produce multifunctional (that is, bifunctional, trifunctional, tetrafunctional, or the like) antibodies.

In the present invention, the "multifunctional" refers to an antibody or antigen-binding fragment that has two or more activities or functions (for example, antigen-binding capacity, enzyme activity, and ligand- or receptor-binding capacity). For example, the antibody of the present invention may be bound to a polypeptide having enzymatic activity, such as luciferase, acetyltransferase, galactosidase, or the like. Multifunctional antibodies also include multivalent or multispecific (that is, bispecific, trispecific, or the like) forms of antibodies.

In addition, the antibody or antigen-binding fragment according to the present invention is capable of binding specifically to an epitope comprising a polypeptide represented by any one of the amino acid sequences of SEQ ID NOs: 35 to 45, which is present on regulatory T cells, thereby suppressing the function of the regulatory T cells and regulating the activity of effector T cells, thus preventing or treating various diseases, such as immune-related disease, neurodegenerative disease, or neuroinflammatory disease.

In the present invention, the "immune-related disease" may be, but is not limited to, at least one selected from the group consisting of autoimmune disease, graft-versus-host disease, organ transplant rejection, asthma, atopy, and acute or chronic inflammatory disease.

In the present invention, the autoimmune disease may be, but is not limited to, at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

The "brain nervous system disease" which is to be prevented, ameliorated or treated by the composition provided in the present invention may be a neurodegenerative disease or a neuroinflammatory disease.

In the present invention, the "neurodegenerative disease" and the "neuroinflammatory disease" may be selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

Another embodiment of the present invention provides an antibody-drug conjugate (ADC) comprising: the binding molecule, antibody or antigen-binding fragment provided in the present invention; and a drug.

In the present invention, the "antibody-drug conjugate (ADC)" refers to a form in which the drug and the antibody are chemically linked to each other without degrading biological activity of the antibody and the drug. In the present invention, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

In the present invention, the "drug" may mean any substance having a certain biological activity for a cell, which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

In the present invention, examples of the reactive group capable of reacting and crosslinking with the α-amine group are not limited to particular types, as long as the reactive group can react and crosslink with an α-amine group at the N-terminus of a heavy or light chain of an antibody. Examples of the reactive group include all types of groups known in the art, which are capable of reacting with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, without being limited thereto.

In the present invention, the antibody-drug conjugate comprises the antibody or antigen-binding fragment that binds specifically to the epitope of the present invention, that is, Lrig-1 protein, or an epitope comprising a polypeptide consisting of a partial amino acid sequence of the extracellular domain of the Lrig-1 protein, or an epitope comprising a polypeptide represented by any one of the amino acid sequences of SEQ ID NOs: 7 to 17. Here, the drug may include any drug that can treat a disease targeted by the antibody that binds specifically to the Lrig-1 protein. For example, the drug may be, but is not limited to, a drug that can treat immune-related disease, or a drug that can treat neurodegenerative disease or neuroinflammatory disease.

Another embodiment of the present invention provides a pharmaceutical composition for preventing or treating various diseases, such as immune-related disease or brain nervous system disease, the pharmaceutical composition comprising, as an active ingredient, the antibody or antigen-binding fragment provided in the present invention, or the antibody-drug conjugate (ADC).

In the present invention, the binding molecule, the antibody or antigen-binding fragment, or the antibody-drug conjugate obtained by conjugating the drug thereto, which is contained as an active ingredient in the pharmaceutical composition, is capable of binding specifically to an epitope comprising a polypeptide represented by any one of the amino acid sequences of SEQ ID NOs: 35 to 45, thereby very effectively treating various diseases through the process of controlling the function of regulatory T cells and regulating the activity of effector T cells.

The "immune-related disease" in the present invention may be, but is not limited to, at least one selected from the group consisting of autoimmune disease, graft-versus-host disease, organ transplant rejection, asthma, atopy, and acute or chronic inflammatory disease.

The autoimmune disease in the present invention may be, but is not limited to, at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

The "brain nervous system disease" which is to be prevented, ameliorated or treated by the composition provided in the present invention may be a neurodegenerative disease or a neuroinflammatory disease.

In the present invention, the "neurodegenerative disease" and the "neuroinflammatory disease" may be selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

Another embodiment of the present invention provides a pharmaceutical composition for preventing or treating immune-related disease or brain nervous system disease comprising, as an active ingredient, a chimeric antigen receptor (CAR) comprising an antigen-specific binding domain, a linking domain, and a CD3 zeta (ζ) signaling domain.

In the present invention, the term "chimeric antigen receptor" or "CAR" refers to an engineered receptor comprising an extracellular antigen-binding domain an intracellular signaling domain. Although the most common type of CAR comprises a single chain variable fragment (scFv) derived from a monoclonal antibody fused to a transmembrane and intracellular domain of a T cell co-receptor such as the CD3 zeta (ζ) chain, the invention described herein is not limited to these domains. Rather, as used herein, "chimeric antigen receptor" or "CAR" refers to any receptor engineered to express any intracellular signaling molecule and an extracellular antigen-binding domain fused or linked to any intracellular signaling molecule. In the present invention, the binding domain may comprise a single-chain variable fragment (scFv) capable of specifically recognizing Lrig-1 protein. In the present invention, the "single-chain variable fragment" or "scFv" refers to a fusion protein of the variable heavy (VH) and variable light (VL) chains of an antibody with a peptide linker between the VL and VH.

In addition, in the present invention, the VH domain and the VL domain may be linked together by a flexible linker. In the present invention, the flexible linker may be a glycine/serine linker of about 10 to 30 amino acids (e.g., 30, 25, 20, 19, 18, 17, 16, 15, 14, 13, 12, 11, 10, 9, 8, 7, 6 or 5 amino acids), preferably 15 amino acids in length. In the present invention, the length of the linker may be an important determinant of the chimeric antigen receptor. Thus, linkers having a shorter length than the lower limit of the above range can enhance affinity but can also lead to intracellular multimer formation, thus impairing expression of the CAR. On the other hand, linkers having a longer length than the upper limit of the above range may decrease antigen affinity by moving the VL and VH CDRs further apart in space.

The chimeric antigen receptor of the present invention may further comprise at least one of a hinge region (or spacer) and a signaling domain. In the present invention, the hinge region is a portion connecting the antigen binding domain and the transmembrane domain, and is also called spacer. The hinge region has the purpose for expanding the antigen binding domain from T cell membrane or NK cell membrane. In the present invention, the hinge region may be obtained, for example, from any suitable sequence from any genus, including human or a part thereof, or may include a hinge region of a human protein including CD8, CD28, 4-1BB, OX40, all or part of CD3 zeta (ζ) chain, T cell receptor α or β chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, which are commonly used in the art, without being limited thereto. In addition, in the present invention, the hinge region may comprise one selected from, but not limited to, immunoglobulin (e.g., IgG1, IgG2, IgG3, IgG4, and IgD).

In the present invention, the signaling domain refers to the portion of the chimeric antigen receptor that is found or is engineered to be found inside a T cell. In the present invention, the signaling domain may or may not also contain a transmembrane domain which functions to anchor the chimeric antigen receptor in the plasma membrane of a T cell. In the present invention, the transmembrane domain and the signaling domain may be derived from the same protein (e.g., CD3ζ molecule). Alternatively, the transmembrane domain and the signaling domain may be derived from different proteins (e.g., the transmembrane domain of a CD28 and the intracellular signaling domain of a CD3ζ molecule, or vice versa).

In the present invention, the transmembrane domain may comprise, for example, a T cell receptor α or β chain, all or part of CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, without being limited thereto. In the present invention, the costimulatory domain may comprise a functional signaling domain derived from a polypeptide comprising a ligand that binds to 4-1BB (CD137), OX40, CD27, CD28, CD30, CD40, PD-1, CD2, CD7, CD258, natural killing group 2 member C (NKG2C), natural killing group 2 member D (NKG2D), B7-H3, CD83, ICAM-1, LFA-1 (CD11a/CD18), or ICOS, active fragments thereof, functional derivatives thereof, or combinations thereof, without being limited thereto.

In the present invention, the signaling domain may comprise a functional signaling domain derived from a polypeptide comprising all or part of CD3 zeta (ζ), common FcR gamma (FcER1G), Fc gamma RIIIa, FcR beta (Fc epsilon R1b), CD3 gamma, CD3 delta, CD3 epsilon, CD79a, CD79b, DNAX-activating protein 10 (DAP10), DNAX-activating protein 12 (DAP12), active fragments thereof, functional derivatives thereof, or combinations thereof, without being limited thereto. Such signaling domains are known in the art.

The "immune-related disease" in the present invention may be, but is not limited to, at least one selected from the group consisting of autoimmune disease, graft-versus-host disease, organ transplant rejection, asthma, atopy, and acute or chronic inflammatory disease.

The autoimmune disease in the present invention may be, but is not limited to, at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

The "brain nervous system disease" which is to be prevented, ameliorated or treated by the composition provided in the present invention may be a neurodegenerative disease or a neuroinflammatory disease.

In the present invention, the "neurodegenerative disease" and the "neuroinflammatory disease" may be selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

Meanwhile, in the prevention, "prevention" may include, without limitation, any act of blocking symptoms of a disease, or suppressing or delaying the symptoms, using the pharmaceutical composition of the present invention.

In addition, in the present invention "treatment" may include, without limitation, any act of ameliorating or beneficially altering symptoms of a disease, using the pharmaceutical composition of the present invention.

In the present invention, the pharmaceutical composition may be characterized by being in the form of capsules, tablets, granules, injections, ointments, powders, or beverages, and the pharmaceutical composition may be characterized by being intended for human subjects.

In the present invention, for use, the pharmaceutical composition may be formulated in the form of oral preparations such as powders, granules, capsules, tablets, and aqueous suspensions, preparations for external use, suppositories, and sterile injectable solutions, according to respective conventional methods, without being limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The pharmaceutical composition of the present invention may be prepared in various dosage forms by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, examples of carriers, diluents, or excipients suitable for making preparations include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like. In addition, a filler, an anti-coagulant, a lubricant, a wetting agent, a fragrance, an emulsifier, a preservative, and the like may further be included.

The route of administration of the pharmaceutical composition of the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including the activity of a certain compound used, the patient's age, body weight, general health status, sex, diet, the time of administration, the route of administration, the rate of excretion, drug combination, and the severity of a certain disease to be prevented or treated. Although the dose of the pharmaceutical composition may vary depending on the patient's condition and body weight, the severity of disease, the drug form, the route of administration, and the duration of administration, it may be appropriately selected by those skilled in the art. The pharmaceutical composition may be administered at a dose of 0.0001 to 50 mg/kg/day or 0.001 to 50 mg/kg/ day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

Another embodiment of the present invention is directed to a method for preventing or treating various diseases such as immune-related disease or brain nervous system disease, the method comprising a step of administering, to a subject, a pharmaceutically effective amount of the binding molecule according to the present invention, or the antibody-drug conjugate (ADC), or the chimeric antigen receptor (CAR).

The "immune-related disease" in the present invention may be, but is not limited to, at least one selected from the group consisting of autoimmune disease, graft-versus-host disease, organ transplant rejection, asthma, atopy, and acute or chronic inflammatory disease.

The autoimmune disease in the present invention may be, but is not limited to, at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

The "brain nervous system disease" which is to be prevented, ameliorated or treated by the composition provided in the present invention may be a neurodegenerative disease or a neuroinflammatory disease.

In the present invention, the "neurodegenerative disease" and the "neuroinflammatory disease" may be selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

The antibody or antigen-binding fragment according to the present invention and the antibody-drug conjugate according to the present invention are capable of binding specifically to an epitope comprising a polypeptide represented by any one of the amino acid sequences of SEQ ID NOs: 35 to 45, thereby controlling the function of regulatory T cells and regulating the activity of effector T cells, thus very effectively preventing or treating various diseases.

In the present invention, the "subject" is a subject suspected of developing immune-related disease or brain nervous system disease. The subject suspected of developing immune-related disease or brain nervous system disease refers to mammals, such as humans, mice, and domestic animals, which had or are likely to develop the disease in question. However, the subject includes, without limitation, any subject treatable with the antibody, antigen-binding fragment or antibody-drug conjugate of the present invention.

The method of the present invention may comprise administering a pharmaceutically effective amount of the antibody or the antibody-drug conjugate. An appropriate total daily amount used may be determined by an attending physician within the scope of sound medical judgment, and administration may be made once or several times. However, for the purposes of the present invention, a specific therapeutically effective amount for a particular patient is preferably applied differently depending on various factors, including the type and degree of reaction to be achieved, the specific composition including whether other agents are used therewith as the case may be, the patient's age, body weight, general health status, sex, and diet, the time of administration, the route of administration, the secretion rate of the composition, the duration of treatment, and drugs used simultaneously or in combination with the specific composition, and similar factors well known in the medical field.

Meanwhile, the method for preventing or treating disease may be, but is not limited to, a combination therapy that further comprises administering a compound or substance having therapeutic activity against one or more diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, the dose of the antibody or antibody-drug conjugate may be, but is not limited to, about 0.0001 µg to 500 mg per kg of patient's body weight.

In the present invention, covalent Labelling MS (CL-MS)and cross-linking MS (XL-MS) experiments were conducted to identify epitopes to which GTC210-01, GTC210-02, GTC210-03, GTC210-04, GTC110-01, GTC110-02, GTC110-03, GTC110-03 mouse antibody, or GTC110-03 humanized antibody can bind. In the two experiments, portions that had identical sequences or were exposed to the outside (percent labeling of 50% or more) could be screened as epitopes of the Lrig-1 protein.

The protein used in the screening method of the present invention is a form displayed on the cell surface, a form displayed on the surface of a virus (e.g., bacteriophage), an isolated form, or a purified form. When a protein displayed on the surface of a cell or virus is used, it is preferable to immobilize the cell or virus on a solid substrate to speed up or automate screening. In addition, it is also preferable to immobilize a protein in isolated or purified form on a solid substrate. Substrates may be any substrates commonly used in the art, and examples thereof include, but are not limited to, hydrocarbon polymers such as polystyrene and polypropylene, glass, metals, and gels. The solid substrate may be provided in the form of a dipstick, a microtiter plate, a particle (e.g., bead), an affinity column, or an immunoblot membrane (e.g., polyvinylidene fluoride membrane). Most preferably, the solid substrate is a microtiter plate.

The screening method of the present invention may be performed in a variety of ways, and in particular, it may be performed in a high-throughput manner according to various binding assay techniques known in the art.

In the screening method of the present invention, the test substance or the proteins may be labeled with a detectable label. Examples of the detectable label include chemical labels (e.g., biotin), enzymatic labels (e.g., horseradish peroxidase, alkaline phosphatase, peroxidase, luciferase, β-galactosidase, and β-glucosidase), radioactive labels (e.g., C¹⁴, I¹²⁵, P³² and S³⁵), fluorescent labels [e.g., coumarin, fluorescein, fluoresein isothiocyanate (FITC), rhodamine 6G, rhodamine B, 6-carboxy-tetramethylrhodamine (TAMRA), Cy-3, Cy-5, Texas Red, Alexa Fluor, DAPI (4,6-diamidino-2phenylindole), HEX, TET, Dabsyl, and FAM], luminescent labels, chemiluminescent labels, fluorescence resonance energy transfer (FRET) labels, or metallic labels (e.g., gold and silver).

When a protein or test substance labeled with a detectable label is used, whether the binding between the protein and the test substance occurred may be analyzed by detecting signals from the label. For example, when alkaline phosphatase is used as the label, signals are detected using a chromogenic substrate such as bromochloroindolyl phosphate (BCIP), nitro blue tetrazolium (NBT), naphthol-AS-B1-phosphate, or ECF (enhanced chemifluorescence). When horseradish peroxidase is used as the label, signals are detected using substrates such as chloronaphthol, aminoethylcarbazole, diaminobenzidine, D-luciferin, lucigenin (bis-N-methylacridinium nitrate), resorufin benzyl ether, luminol, Amplex Red (10-acetyl-3,7-dihydroxyphenoxazine), HYR (p-phenylenediamine-HCl and pyrocatechol), tetramethylbenzidine (TMB), 2,2'-azino-bis(3-ethylbenzthiazoline sulfonate (ABTS), o-phenylenediamine (OPD), or naphthol/pyronine.

One embodiment of the present invention provides an epitope of Lrig-1 (leucine-rich and immunoglobulin-like domains 1) protein comprising at least any one polypeptide selected from the group consisting of polypeptides consisting of the amino acid sequences represented by SEQ ID NO: 35 to SEQ ID NO: 45.

Another embodiment of the present invention provides the epitope wherein the polypeptide consists of the amino acid sequence represented by SEQ ID NO: 38 or 44.

Still another embodiment of the present invention provides the epitope wherein the polypeptide consists of the amino acid sequences represented by SEQ ID NOs: 35 to 37, 39 to 43 and 45.

Yet another embodiment of the present invention provides a nucleic acid molecule encoding the epitope.

Still yet another embodiment of the present invention provides an expression vector into which the nucleic acid molecule has been inserted.

A further embodiment of the present invention provides a host cell line transfected with the expression vector.

Another further embodiment of the present invention provides a binding molecule that binds specifically to an epitope comprising at least any one polypeptide selected from the group consisting of polypeptides consisting of the amino acid sequences represented by SEQ ID NO: 35 to SEQ ID NO: 45.

Still another further embodiment of the present invention provides the binding molecule which is an antibody or a fragment thereof.

Yet another further embodiment of the present invention provides the binding molecule wherein the antibody is a chimeric antibody, a humanized antibody, a bivalent-bispecific molecule, a minibody, a domain antibody, a bispecific antibody, an antibody mimic, a diabody, a triabody, a tetrabody, or a fragment thereof.

Still yet another further embodiment of the present invention provides a chimeric antigen receptor (CAR) comprising an antigen-specific binding domain, a linking domain, and a CD3 zeta (ζ) signaling domain, wherein the antigen-specific binding domain is a fusion protein comprising: an antigen-specific binding domain that binds specifically to an epitope comprising at least any one polypeptide selected from the group consisting of polypeptides consisting of the amino acid sequences represented by SEQ ID NO: 35 to SEQ ID NO: 45; and an immunoglobulin Fc region.

Another embodiment of the present invention provides an antibody-drug conjugate comprising the binding molecule and a drug.

Still another embodiment of the present invention provides a pharmaceutical composition for preventing or treating immune-related disease comprising, as an active ingredient, any one of the binding molecule, the chimeric antigen receptor, and the antibody-drug conjugate.

Yet another embodiment of the present invention provides the pharmaceutical composition for preventing or treating immune-related disease, wherein the immune-related disease is at least one selected from the group consisting of autoimmune disease, graft-versus-host disease, organ transplant rejection, asthma, atopy, and acute or chronic inflammatory disease.

Still yet another embodiment of the present invention provides a pharmaceutical composition for preventing or treating brain nervous system disease comprising, as an active ingredient, any one of the binding molecule, the chimeric antigen receptor, and the antibody-drug conjugate.

A further embodiment of the present invention provides the pharmaceutical composition for preventing or treating brain nervous system disease, wherein the brain nervous system disease is neurodegenerative disease or neuroinflammatory disease.

Another further embodiment of the present invention provides the pharmaceutical composition for preventing or treating neurodegenerative disease or neuroinflammatory disease, wherein the neurodegenerative disease or neuroinflammatory disease is at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.

Still another further embodiment of the present invention provides a method for screening an epitope of Lrig-1 (leucine-rich and immunoglobulin-like domains 1) protein comprising steps of (a) producing an antibody comprising a heavy-chain region represented by the amino acid sequence of SEQ ID NO: 17 and a light-chain region represented by the amino acid sequence of SEQ ID NO: 18, or producing an antibody comprising a heavy-chain region represented by the amino acid sequence of SEQ ID NO: 19 and a light-chain region represented by the amino acid sequence of SEQ ID NO: 20, or producing an antibody comprising a heavy-chain region represented by the amino acid sequence of SEQ ID NO: 21 and a light-chain region represented by the amino acid sequence of SEQ ID NO: 22, or producing an antibody comprising a heavy-chain region represented by the amino acid sequence of SEQ ID NO: 23 and a light-chain region represented by the amino acid sequence of SEQ ID NO: 24, or producing an antibody comprising a heavy-chain region represented by the amino acid sequence of SEQ ID NO: 25 and a light-chain region represented by the amino acid sequence of SEQ ID NO: 26, or producing an antibody comprising a heavy-chain region represented by the amino acid sequence of SEQ ID NO: 27 and a light-chain region represented by the amino acid sequence of SEQ ID NO: 28, or producing an antibody comprising a heavy-chain region represented by the amino acid sequence of SEQ ID NO: 29 and a light-chain region represented by the amino acid sequence of SEQ ID NO: 30, or producing an antibody comprising a heavy-chain region represented by the amino acid sequence of SEQ ID NO: 31 and a light-chain region represented by the amino acid sequence of SEQ ID NO: 32, or producing an antibody comprising a heavy-chain region represented by the amino acid sequence of SEQ ID NO: 33 and a light-chain region represented by the amino acid sequence of SEQ ID NO: 34, and (b) screening an epitope of Lrig-1 (leucine-rich and immunoglobulin-like domains 1) protein using the antibody through a covalent labelling MS (CL-MS) or cross-linking MS (XL-MS) experiment.

Yet another further embodiment of the present invention provides a method of screening an epitope of Lrig-1protein using the antibody.

Another embodiment of the present invention provides a method of screening a binding molecule, which is an antibody or a fragment thereof, using the epitope.

Still another embodiment of the present invention provides a method of binding the binding molecule, which is an antibody or a fragment thereof, to the epitope.

Yet another embodiment of the present invention provides a binding molecule, which is an antibody or a fragment thereof that binds specifically to Lrig-1 (leucine-rich and immunoglobulin-like domains 1) protein, the binding molecule comprising:
(a) a heavy-chain variable region (VH) comprising: a heavy-chain variable region (VH) CDR1 selected from the group consisting of the amino acid sequences represented by SEQ ID NOs: 46, 52, 58, 64, 70, 76, 82 and 88; a heavy-chain variable region (VH) CDR2 selected from the group consisting of the amino acid sequences represented by SEQ ID NOs: 47, 53, 59, 65, 71, 77, 83 and 89; and a heavy-chain variable region (VH) CDR3 selected from the group consisting of the amino acid sequences represented by SEQ ID NOs: 48, 54, 60, 66, 72, 78, 84 and 90; and
(b) a light-chain variable region (VL) comprising: a light-chain variable region (VL) CDR1 selected from the group consisting of the amino acid sequences represented by SEQ ID NOs: 49, 55, 61, 67, 73, 79, 85 and 91; a light-chain variable region (VL) CDR2 selected from the group consisting of the amino acid sequences represented by SEQ ID NOs: 50, 56, 62, 68, 74, 80, 86 and 92; and a light-chain variable region (VL) CDR3 selected from the group consisting of the amino acid sequences represented by SEQ ID NOs: 51, 57, 63, 69, 75, 81, 87 and 93,
wherein the binding molecule binds specifically to an epitope comprising at least any one polypeptide selected from the group consisting of polypeptides consisting of the amino acid sequences represented by SEQ ID NO: 35 to SEQ ID NO: 45.

### Advantageous Effects

The antibody or antigen-binding fragment that binds specifically to the epitope of Lrig-1 protein according to the present invention is capable of binding specifically to the epitope of the present invention, which is present on regulatory T cells, thereby suppressing the function of the regulatory T cells and maintaining or increasing the activity of effector T cells. Thus, the antibody or the antigen-binding fragment may be very efficiently used to prevent, ameliorate or treat various diseases.

### Brief Description of Drawings

FIG. 1 shows the structure of Lrig-1 protein according to an embodiment of the present invention.
FIG. 2 shows the structure of Lrig-1 protein according to an embodiment of the present invention.
FIG. 3 shows the expression level of Lrig-1 mRNA according to an embodiment of the present invention.
FIG. 4 shows the expression level of Lrig-1 mRNA according to an embodiment of the present invention.
FIG. 5 shows the expression level of Lrig-1 mRNA according to an embodiment of the present invention.
FIG. 6 shows the expression level of Lrig-1, Lrig-2 and Lrig-3 mRNAs according to an embodiment of the present invention.
FIG. 7 shows the results of comparing expression levels of Lrig-1 protein in regulatory T cells and non-regulatory T cells according to an embodiment of the present invention.
FIG. 8 shows expression of Lrig-1 protein on the surface of regulatory T cells according to an embodiment of the present invention.
FIG. 9 shows the results of analyzing the binding capacities of antibodies (GTC210-01, GTC210-02, GTC210-03, GTC210-04, GTC110-01, GTC110-02, GTC110-03 and GTC110-04) to Lrig-1 protein in an embodiment of the present invention.
FIG. 10 shows the results of analyzing the mechanisms of regulating Lrig-1 protein-induced Stat3 phosphorylation in regulatory T cells by Lrig-1 protein-specific monoclonal antibodies (GTC210-01, GTC210-02, GTC210-03, GTC210-04, GTC110-01, GTC110-02, GTC110-03 and GTC110-04) in an embodiment of the present invention.
FIG. 11 shows the results of checking weight loss after intraperitoneally injecting the monoclonal antibodies according to the present invention into inflammatory bowel disease animal models according to an embodiment of the present invention.
FIG. 12 shows an experimental design for producing a multiple sclerosis animal model according to an embodiment of the present invention.
FIG. 13 is a graph showing the therapeutic effect evaluation score obtained by injecting the monoclonal antibody according to the present invention into a multiple sclerosis animal model according to an embodiment of the present invention.
FIG. 14 is a graph showing the therapeutic effect evaluation score obtained by injecting the monoclonal antibody according to the present invention into a multiple sclerosis animal model according to an embodiment of the present invention.
FIG. 15 shows that demyelination was noticeably reduced when the antibody according to an embodiment of the present invention was administered.
FIG. 16 shows that expression of T helper cell 1 (CD4+ T-bet+) and T helper cell 2 decreased, expression of T helper cell 17 (CD4+ RoRyt+) and regulatory T cell (CD4+ Foxp3+) increased, expression of various inflammatory cytokines (IFN-gamma, IL-17A) decreased, and expression of anti-inflammatory cytokine (IL-10) increased.
FIGS. 17 to 20 show the results of evaluating the therapeutic effect of injection of the monoclonal antibody according to the present invention in groups (G1 to G4) of Alzheimer's animal models through a Y-maze test (FIG. 17), a novel object recognition test (FIG. 18) and a water maze test (FIGS. 19 and 20), according to an embodiment of the present invention.
FIGS. 21 to 23 show the results of covalent labeling mass spectrometry of peptides degraded by treatment with proteases (chymotrypsin (FIG. 21), trypsin (FIG. 22), and Asp-N/Lys-C (FIG. 23)) according to an embodiment of the present invention.

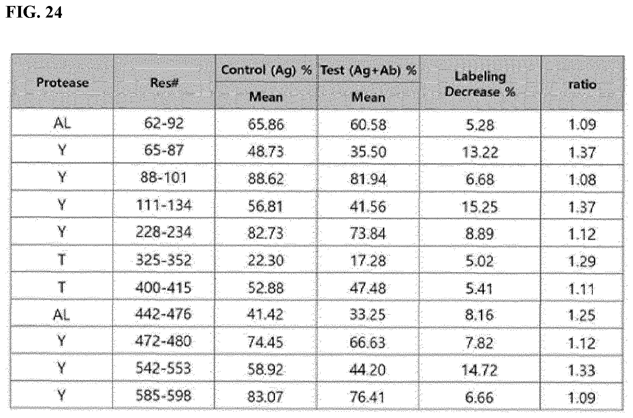

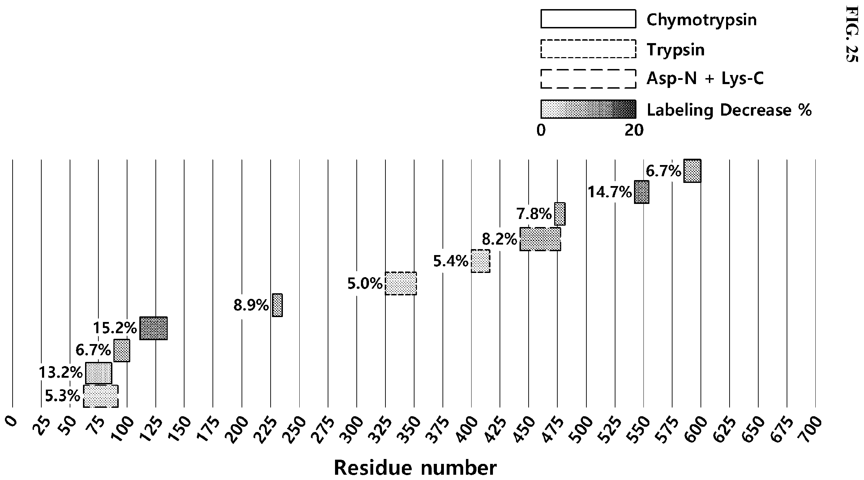

FIGS. 24 and 25 show the results of performing alignment and comparison based on the sequences identified by covalent labeling mass spectrometry of the peptides degraded by each protease according to an embodiment of the present invention.

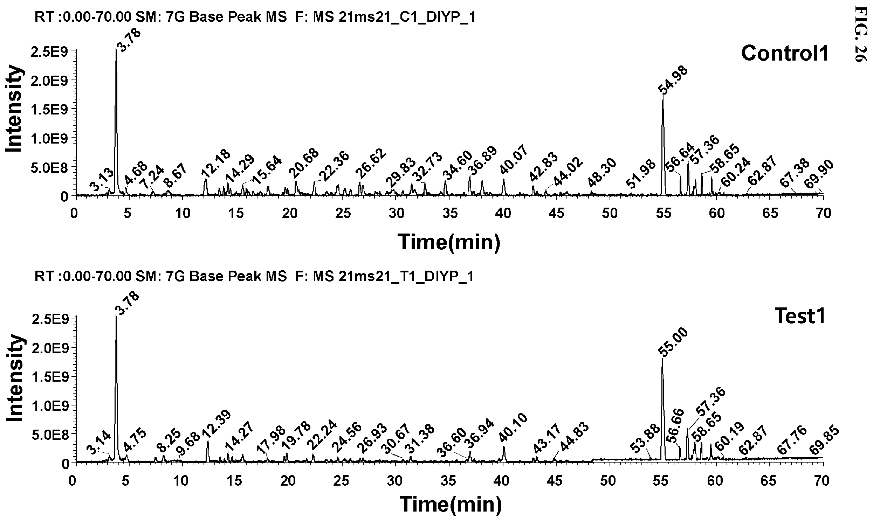

FIGS. 26 and 27 show the results of cross-linking assay of peptides degraded by treatment with proteases (chymotrypsin (FIG. 26) and trypsin (FIG. 27)) according to an embodiment of the present invention.
FIGS. 28 and 29 show the results of performing alignment and comparison based on the sequences identified by cross-linking assay of the peptides degraded by each protease according to an embodiment of the present invention.
FIG. 30 shows the results of aligning and analyzing epitopes identified by covalent labeling mass spectrometry and cross-linking assay according to an embodiment of the present invention.

### Best Mode

The present invention is directed to an epitope of Lrig-1 (leucine-rich and immunoglobulin-like domains 1) protein, which is an antigen present on the surface of a regulatory T cell, and an antibody or antigen-binding fragment that binds specifically thereto.

An embodiment of the present invention provides the epitope of Lrig-1 (leucine-rich and immunoglobulin-like domains 1), which is selected from the group consisting of polypeptides comprising the amino acid sequences represented by SEQ ID NOs: 38, 39, 41, 42, 44 and 45.

An embodiment of the present invention provides a binding molecule that binds specifically to an epitope comprising at least any one polypeptide selected from the group consisting of polypeptides consisting of the amino acid sequences represented by SEQ ID NOs: 35 to 45.

An embodiment of the present invention provides a chimeric antigen receptor (CAR) comprising an antigen-specific binding domain, a linking domain, and a CD3 zeta (ζ) signaling domain, wherein the antigen-specific binding domain is a fusion protein comprising: an antigen-specific binding domain that binds specifically to an epitope comprising at least any one polypeptide selected from the group consisting of polypeptides consisting of the amino acid sequences represented by SEQ ID NO: 35 to SEQ ID NO: 45; and an immunoglobulin Fc region.

An embodiment of the present invention provides a binding molecule, which is an antibody or a fragment thereof that binds specifically to Lrig-1 (leucine-rich and immunoglobulin-like domains 1) protein, the binding molecule comprising:
(a) a heavy-chain variable region (VH) comprising: a heavy-chain variable region (VH) CDR1 selected from the group consisting of the amino acid sequences represented by SEQ ID NOs: 46, 52, 58, 64, 70, 76, 82 and 88; a heavy-chain variable region (VH) CDR2 selected from the group consisting of the amino acid sequences represented by SEQ ID NOs: 47, 53, 59, 65, 71, 77, 83 and 89; and a heavy-chain variable region (VH) CDR3 selected from the group consisting of the amino acid sequences represented by SEQ ID NOs: 48, 54, 60, 66, 72, 78, 84 and 90; and
(b) a light-chain variable region (VL) comprising: a light-chain variable region (VL) CDR1 selected from the group consisting of the amino acid sequences represented by SEQ ID NOs: 49, 55, 61, 67, 73, 79, 85 and 91; a light-chain variable region (VL) CDR2 selected from the group consisting of the amino acid sequences represented by SEQ ID NOs: 50, 56, 62, 68, 74, 80, 86 and 92; and a light-chain variable region (VL) CDR3 selected from the group consisting of the amino acid sequences represented by SEQ ID NOs: 51, 57, 63, 69, 75, 81, 87 and 93,
wherein the binding molecule binds specifically to an epitope comprising at least any one polypeptide selected from the group consisting of polypeptides consisting of the amino acid sequences represented by SEQ ID NO: 35 to SEQ ID NO: 45.

### Mode for Invention

Hereinafter, the present invention will be described in more detail with reference to examples. These examples are only for illustrating the present invention in more detail, and it will be obvious to those of ordinary skill in the art that the scope of the present invention according to the subject matter of the present invention is not limited by these examples.

### Examples

### [Preparation Example 1] T Cell Subset Culture

In order to identify whether the Lrig-1 protein is expressed only in regulatory T cells (Treg) cells, Th0, Th1, Th2, Th17 and iTreg, which are T cell subsets, were prepared. The iTreg refers to cells whose differentiation has been artificially induced in a medium having the following composition, unlike nTreg which has been naturally isolated.

The T cell subsets were obtained by isolating naive T cells from mouse spleens, and then inducing the isolated cells to differentiate into each T cell subset by 72 hours of culture in RPMI1640 (Invitrogen Gibco, Grand Island, NY) nutrient medium, which contained 10% fetal bovine serum (FBS; hyclone, logan, UT) and further contained the components shown in Table 1 below, in an incubator at 37°C under 5% CO₂.

**[Table 1]**

| Differentiated cells | Composition |
|---|---|
| Th0 | anti-CD3 and anti-CD28 |
| Th1 | IL-12 and anti-IL-4 antibodies |
| Th2 | IL-4 and anti-IFNβ |
| Th17 | IL-6, TGFβ, anti-IFNβ and anti-IL-4 |
| iTreg | IL-2 and TGFβ |

### [Example 1] Structural Analysis of Lrig-1

The three-dimensional steric structure of the extracellular domain of Lrig-1 protein was predicted to produce an antibody specific for the Lrig-1 protein, a protein present on the surfaces of regulatory T cells.

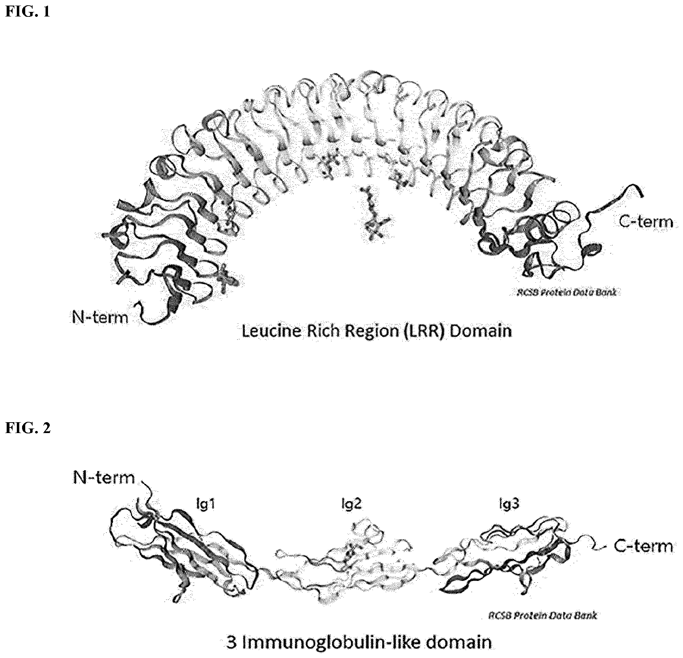

First, in order to predict the amino acid sequences of epitopes, tools of Uniprot (http://www.uniprot.org) and RCSB Protein Data Bank (http://www.rcsb.org/pdb) were used to predict the three-dimensional steric structure of the extracellular domain (ECD) of the Lrig-1 protein, and then the results are shown in FIGS. 1 and 2.

As shown in FIG. 1, a total of 15 leucine-rich regions (LRR1 to LRR15) were present in the Lrig-LRR domain (amino acid sequence at positions 41 to 494) in the extracellular domain of the Lrig-1 protein. Each of the LRR domains consisted of 23 to 27 amino acids, with 3 to 5 leucine residues being present.

In addition, as shown in FIG. 2, three immunoglobulin-like domains were present at positions 494 to 781 in the amino acid sequence of the Lrig-1 protein in the extracellular domain of the Lrig-1 protein.

### [Example 2] Identification of Specific Expression of Lrig-1 mRNA in Regulatory T Cells

It was verified whether the Lrig-1 protein could act as a biomarker specific for regulatory T cells.

For the verification, CD4⁺ T cells were isolated from mouse spleens by magnet-activated cell sorting (MACS) using CD4 beads. Thereafter, regulatory T (CD4⁺CD25⁺ T) cells and non-regulatory T (CD4⁺CD25⁻ T) cells were isolated by fluorescence activated cell sorting (FACS) using CD25 antibody. For the respective cells and the cells differentiated in Preparation Example 1, mRNA was extracted using Trizol, and then genomic DNA was removed from genomic RNA using a gDNA extraction kit (Qiagen) according to the protocol provided by the manufacturer. The mRNA from which gDNA has been removed was synthesized into cDNA using the BDsprint cDNA synthesis kit (Clonetech).

Real-time polymerase chain reaction (RT PCR) was performed to quantitatively analyze the expression level of Lrig-1 mRNA in the cDNA.

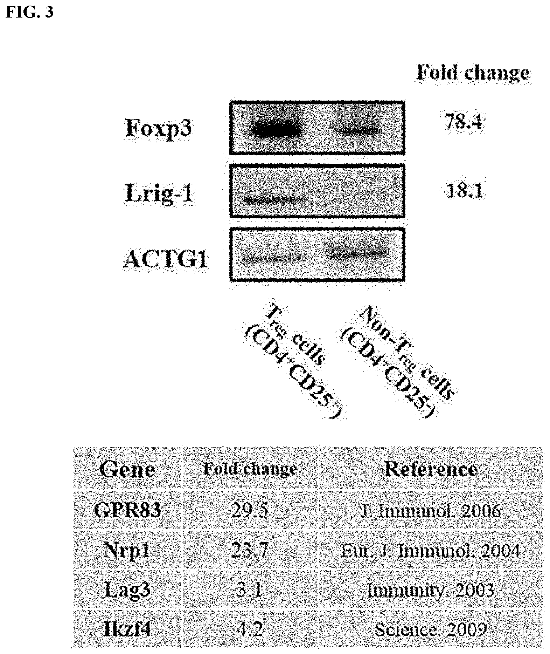

The real-time polymerase chain reaction was performed with primers shown in Table 2 below using SYBR Green (Molecular Probes) according to the protocol provided by the manufacturer for 40 cycles, each consisting of 95°C for 3 minutes, 61°C for 15 seconds, and 72°C for 30 seconds. The relative gene expression level was calculated using the ΔCT method and normalized using HPRT. The results are shown in FIGS. 3 to 6.

**[Table 2]**

| Primer | Direction | SEQ ID NO. |
|---|---|---|
| Mouse Lrig-1 | Forward | SEQ ID NO: 7 |
| | Reverse | SEQ ID NO: 8 |
| Mouse Lrig-2 | Forward | SEQ ID NO: 9 |
| | Reverse | SEQ ID NO: 10 |
| Mouse Lrig-3 | Forward | SEQ ID NO: 11 |
| | Reverse | SEQ ID NO: 12 |
| Mouse FOXP3 | Forward | SEQ ID NO: 13 |
| | Reverse | SEQ ID NO: 14 |
| ACTG1 | Forward | SEQ ID NO: 15 |
| | Reverse | SEQ ID NO: 16 |

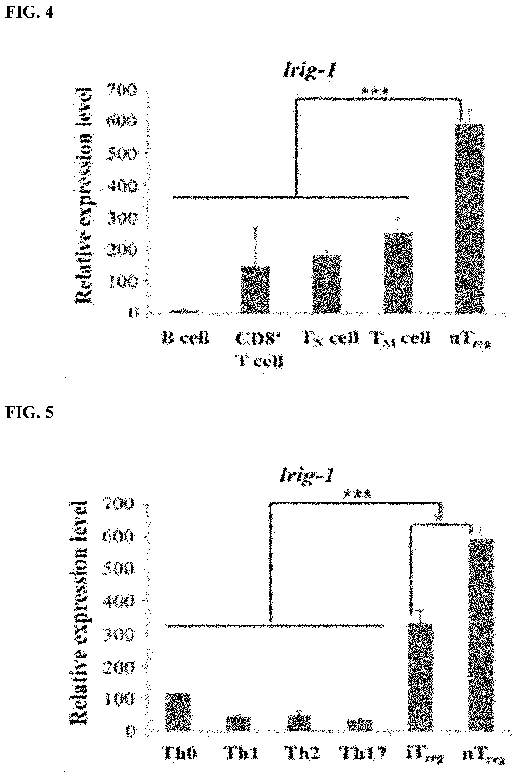

As shown in FIG. 3, it can be seen that the expression level of Lrig-1 in the regulatory T (CD4⁺CD25⁺ T) cells was 18.1 times higher than that in the non-regulatory T (CD4⁺CD25⁻ T) cells. This was an about 10 times higher expression level than those of Lag3 and Ikzf4, which are previously known markers for regulatory T cells. In addition, as shown in FIGS. 4 and 5, the expression level of Lrig-1 mRNA was remarkably high in the regulatory T cells compared to other types of immune cells, and in particular, was remarkably high in naturally isolated regulatory T cells (nTreg) compared to induced regulatory T cells (iTreg).

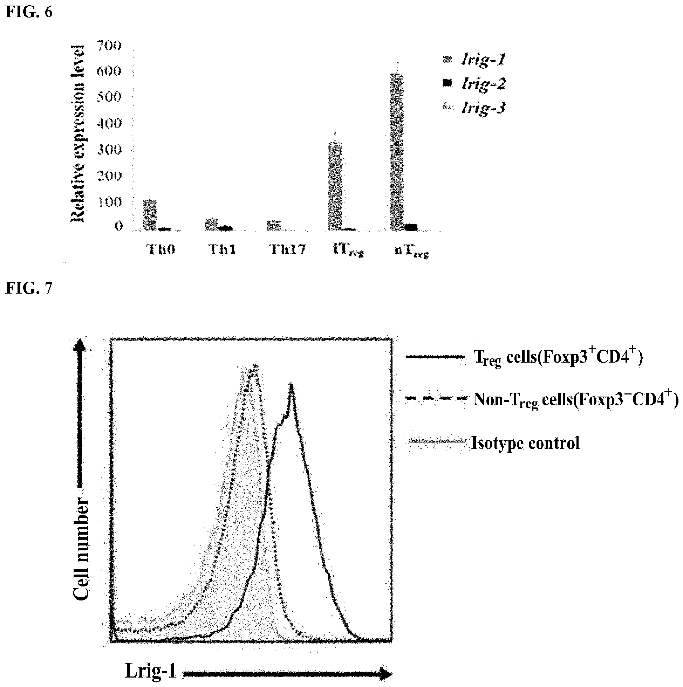

In addition, as shown in FIG. 6, the expression level of Lrig-1 was the highest among the expression levels of Lrig-1, Lrig-2 and Lrig-3 belonging to the Lrig family.

From the above results, it can be seen that the Lrig-1 protein according to the present invention is expressed specifically in regulatory T cells, in particular, naturally occurring regulatory T cells.

### [Example 3] Identification of Specific Expression of Lrig-1 Protein in Regulatory T Cells

It was identified whether the Lrig-1 protein expressed from Lrig-1 mRNA was specifically expressed only in regulatory T cells.

Using FOXP3-RFP-knocked-in mice having red fluorescence protein (RFP) conjugated to the FOXP3 promoter, which is a transcription factor specific for regulatory T cells, CD4⁺ T cells were isolated from the mouse spleens by magnet-activated cell sorting (MACS) using CD4 beads. Subsequently, regulatory T (CD4⁺RFP⁺ T) cells and non-regulatory T (CD4⁺RFP⁻ T) cells were isolated by fluorescence-activated cell sorting (FACS) using RFP protein. The respective cells were stained with the purchased Lrig-1 antibody, a negative control was stained with an isotype antibody, and the expression levels of Lrig-1 therein were measured by fluorescence-activated cell sorting. The results are shown in FIG. 7.

As shown in FIG. 7, the non-regulatory T cells indicated by the dotted line showed almost the same expression level of Lrig-1 as the negative control, whereas there were a large number of cells with a high expression level of Lrig-1 in the regulatory T cells.

From the above results, it can be seen that the Lrig-1 protein according to the present invention is expressed specifically in regulatory T cells.

### [Example 4] Identification of Specific Expression of Lrig-1 Protein on Surface of Regulatory T Cells

In order to be a target of cell therapy, the Lrig-1 protein must be expressed on the surfaces of regulatory T cells, which in turn allows target therapy to be more effectively achieved. Thus, it was identified whether the Lrig-1 protein would be expressed on the surfaces of the regulatory T cells.

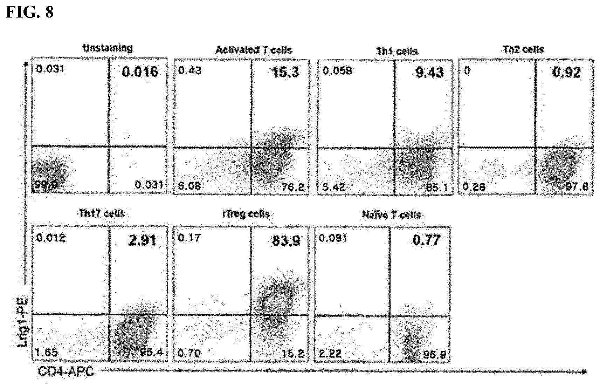

The respective differentiated T cell subsets of Preparation Example 1 were stained with anti-CD4-APC and anti-Lrig-1-PE antibodies, and the expression levels of Lrig-1 on the respective cell surfaces were measured using a fluorescence-activated cell sorter (FACS). The results are illustrated in FIG. 8.

As shown in FIG. 8, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is expressed at a higher level, in particular, on the surface of the Treg cells.

### [Preparation Examples 1 to 8] Preparation of Monoclonal Antibodies Specific to Lrig-1 protein

Antibodies specific to the Lrig-1 protein according to the present invention were produced. These antibodies were not produced by specifying a certain epitope, but were produced as antibodies capable of binding to any site on the Lrig-1 protein.

In order to produce the antibodies, cells expressing the Lrig-1 protein were produced. More specifically, a DNA fragment corresponding to SEQ ID NO: 2 and pcDNA (hygro) were cleaved with a cleavage enzyme, incubated at 37°C, and ligated to produce pcDNA into which the DNA sequence of the Lrig-1 protein has been inserted. The produced pcDNA into which SEQ ID NO: 2 has been inserted was introduced through transfection into L cells so that the Lrig-1 protein could be expressed on the surface of the L cells.

Light and heavy chain amino acid sequences capable of binding to Lrig-1 expressed on the cell surface were selected from the Human scFv library, thereby selecting a total of eight heavy and light chains.

The selected heavy and light chain amino acid sequences were fused with the mlgG2a Fc region or the human IgG1 Fc region, thereby producing monoclonal antibodies. The sequences of the monoclonal antibodies are shown in Table 3 below.

**[Table 3]**

| Classification | Clone | Location | Sequence information |
|---|---|---|---|
| Preparation Example 1 | GTC210-01 clone | Heavy chain | SEQ ID NO: 17 |
| | | Heavy chain CDR1 | SEQ ID NO: 46 |
| | | Heavy chain CDR2 | SEQ ID NO: 47 |
| | | Heavy chain CDR3 | SEQ ID NO: 48 |
| | | Light chain | SEQ ID NO: 18 |
| | | Light chain CDR1 | SEQ ID NO: 49 |
| | | Light chain CDR2 | SEQ ID NO: 50 |
| | | Light chain CDR3 | SEQ ID NO: 51 |
| Preparation Example 2 | GTC210-02 clone | Heavy chain | SEQ ID NO: 19 |
| | | Heavy chain CDR1 | SEQ ID NO: 52 |
| | | Heavy chain CDR2 | SEQ ID NO: 53 |
| | | Heavy chain CDR3 | SEQ ID NO: 54 |
| | | Light chain | SEQ ID NO: 20 |
| | | Light chain CDR1 | SEQ ID NO: 55 |
| | | Light chain CDR2 | SEQ ID NO: 56 |
| | | Light chain CDR3 | SEQ ID NO: 57 |
| Preparation Example 3 | GTC210-03 clone | Heavy chain | SEQ ID NO: 21 |
| | | Heavy chain CDR1 | SEQ ID NO: 58 |
| | | Heavy chain CDR2 | SEQ ID NO: 59 |
| | | Heavy chain CDR3 | SEQ ID NO: 60 |
| | | Light chain | SEQ ID NO: 22 |
| | | Light chain CDR1 | SEQ ID NO: 61 |
| | | Light chain CDR2 | SEQ ID NO: 62 |
| | | Light chain CDR3 | SEQ ID NO: 63 |
| Preparation Example 4 | GTC210-04 clone | Heavy chain | SEQ ID NO: 23 |
| | | Heavy chain CDR1 | SEQ ID NO: 64 |
| | | Heavy chain CDR2 | SEQ ID NO: 65 |
| | | Heavy chain CDR3 | SEQ ID NO: 66 |
| | | Light chain | SEQ ID NO: 24 |
| | | Light chain CDR1 | SEQ ID NO: 67 |
| | | Light chain CDR2 | SEQ ID NO: 68 |
| | | Light chain CDR3 | SEQ ID NO: 69 |
| Preparation Example 5 | GTC110-01 clone | Heavy chain | SEQ ID NO: 25 |
| | | Heavy chain CDR1 | SEQ ID NO: 70 |
| | | Heavy chain CDR2 | SEQ ID NO: 71 |
| | | Heavy chain CDR3 | SEQ ID NO: 72 |
| | | Light chain | SEQ ID NO: 26 |
| | | Light chain CDR1 | SEQ ID NO: 73 |
| | | Light chain CDR2 | SEQ ID NO: 74 |
| | | Light chain CDR3 | SEQ ID NO: 75 |
| Preparation Example 6 | GTC 110-02 clone | Heavy chain | SEQ ID NO: 27 |
| | | Heavy chain CDR1 | SEQ ID NO: 76 |
| | | Heavy chain CDR2 | SEQ ID NO: 77 |
| | | Heavy chain CDR3 | SEQ ID NO: 78 |
| | | Light chain | SEQ ID NO: 28 |
| | | Light chain CDR1 | SEQ ID NO: 79 |
| | | Light chain CDR2 | SEQ ID NO: 80 |
| | | Light chain CDR3 | SEQ ID NO: 81 |
| Preparation Example 7 | GTC110-03 clone | Heavy chain | SEQ ID NO: 29 |
| | | Heavy chain CDR1 | SEQ ID NO: 82 |
| | | Heavy chain CDR2 | SEQ ID NO: 83 |
| | | Heavy chain CDR3 | SEQ ID NO: 84 |
| | | Light chain | SEQ ID NO: 30 |
| | | Light chain CDR1 | SEQ ID NO: 85 |
| | | Light chain CDR2 | SEQ ID NO: 86 |
| | | Light chain CDR3 | SEQ ID NO: 87 |
| Preparation Example 8 | GTC 110-04 mouse antibody | Heavy chain | SEQ ID NO: 31 |
| | | Heavy chain CDR1 | SEQ ID NO: 88 |
| | | Heavy chain CDR2 | SEQ ID NO: 89 |
| | | Heavy chain CDR3 | SEQ ID NO: 90 |
| | | Light chain | SEQ ID NO: 32 |
| | | Light chain CDR1 | SEQ ID NO: 91 |
| | | Light chain CDR2 | SEQ ID NO: 92 |
| | | Light chain CDR3 | SEQ ID NO: 93 |
| Preparation Example 9 | GTC 110-04 humanized antibody | Heavy chain | SEQ ID NO: 33 |
| | | Heavy chain CDR1 | SEQ ID NO: 88 |
| | | Heavy chain CDR2 | SEQ ID NO: 89 |
| | | Heavy chain CDR3 | SEQ ID NO: 90 |
| | | Light chain | SEQ ID NO: 34 |
| | | Light chain CDR1 | SEQ ID NO: 91 |
| | | Light chain CDR2 | SEQ ID NO: 92 |
| | | Light chain CDR3 | SEQ ID NO: 93 |

### [Example 5] Evaluation of Binding Capacities of Antibodies According to the Present Invention to Lrig-1 Protein

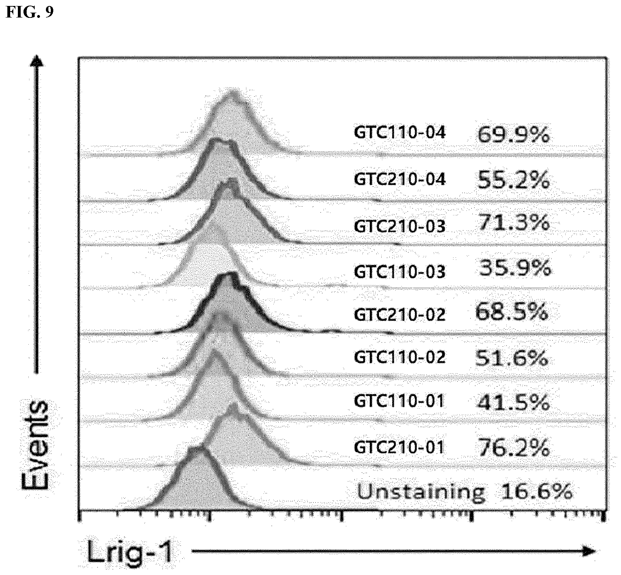

In order to identify whether the monoclonal antibodies according to the present invention, produced in Preparation Examples 1 to 9, well recognize Lrig-1, each of the antibodies of Preparation Examples 1 to 9 was bound to L cells that stably express Lrig-1. Then, an eFlour 670-conjugated secondary antibody capable of recognizing the mouse antibodies was added thereto, and then the binding capacities of the monoclonal antibodies of the Preparation Examples to the Lrig-1 protein were analyzed using FACS. The results are shown in FIG. 9.

As shown in FIG. 9, it could be found that all of the Lrig-1 protein-specific monoclonal antibodies according to the present invention effectively recognized and bound to the Lrig-1 protein present on the surface of L cells.

### [Example 6] Acceptable Changes in CDRs of Antibodies According to the Present Invention

In order to investigate the role of each amino acid position in each CDR, the CDRs of the antibodies of Preparation Example 1 to 9, which bind specifically to the Lrig-1 protein, were listed based on GTC210-03 and analyzed.

Acceptable changes in the CDRs of the antibodies were analyzed. As a result, Table 4 below summarizes acceptable changes in the CDRs of the antibodies that specifically bind to an epitope of Lrig-1 and are effective against immune-related disease by regulating signaling pathways in regulatory T cells, compared to the control, and Table 5 below shows the specific sequences of the CDRs.

**[Table 4] Summary of acceptable changes in CDRs**

| CDR | Position | Acceptable change |
|---|---|---|
| VH CDR1 | S1 | G, N, D |
| | D3 | Y, A |
| VH CDR2 | G1 | L, S, W, A, V |
| | S3 | Y |
| | P4 | H |
| | D5 | G, S |
| | G6 | S, D, |
| | S7 | G |
| | N8 | S |
| | I9 | K, T |
| VH CDR3 | V1 | G, D |
| | G2 | L, I |
| | L3 | G, S |
| | R4 | L, P, N |
| | R6 | K, P, H |
| | Y7 | T, W, L |
| | E8 | G |
| | A9 | L, R, V |
| | S11 | Y |
| | A12 | Y, D, S |
| | Y13 | D, N |
| | G14 | A |
| VL CDR1 | S1 | T |
| | G2 | D |
| | S9 | N |
| | Y11 | N, S, T, D |
| | S13 | T, N, Y |
| VL CDR2 | S1 | D, A |
| | D2 | N |
| | S3 | N |
| | H4 | Q, N |
| VL CDR3 | A1 | G |
| | T2 | S, A |
| | S5 | Y, D |
| | N8 | S |
| | G9 | A |

**[Table 5] Acceptable sequences of CDRs**

| CDR | Sequence | Changed residue | SEQ ID NO. |
|---|---|---|---|
| VH CDR1 | SYDMS | - | 58 |
| | GYDMS | S1→G | 46 |
| | NYYMS | S1→N, D3→Y | 52 |
| | NYDMS | S1→N | 64 |
| | DYDMS | S1→D | 70 |
| | DYYMS | S1→D | 76 |
| | NYAMS | S1→N, D3→A | 82 |
| | NYAMS | S1→N, D3→A | 88 |
| VH CDR2 | GISPDGSNIYYADSVKG | - | 59 |
| | LIYPDSGNKYYADSVK G | G1→L, S3→Y, G6→S, S7→G, I9→K | 47 |
| | GISPGDSSTYY ADSVK G | D5→G, G6→D, N8→S, I9→T | 53 |
| | SISPSSGSIYYADSVKG | G1→S, D5→S, G6→S, S7→G, N8→S | 65 |
| | WISHGGGSIYYADSVK G | G1→W, P4→H, D5→G, S7→G, N8→S | 71 |
| | GISHDSGSKYYADSVK G | P4→H, G6→S, S7→G, N8→S, I9→K | 77 |
| | AIYPGGGSIYYADSVK G | G1→A, S3→Y, D5→G, S7→G, N8→S | 83 |
| | VISHGGGSTYYADSVK G | G1→V, P4→H, D5→G, S7→G, N8→S, I9→T | 89 |
| VH CDR3 | VGLRCRYEACSYAYG MDV | - | 60 |
| | GLGLCKTGLCYYYDA MDV | V1→G, G2→L, L3→G, R4→L, R6→K, Y7→T, E8→G, A9→L, S11→Y, A13→Y, Y14→D, G15→A | 72 |
| | DILPCPWGRCYYDYA MDV | V1→D, G2→I, R4→P, Y7→W, E8→G, A9→R, S11→Y, A13→D, G15→A | 84 |
| | VISNCHLGVCYYSNGM DV | G2→I, L3→S, R4→N, R6→H, Y7→L, E8→G, A9→V, S11→Y, A13-S, Y14→N | 90 |
| | HWTTFDY | - | 78 |
| | DAGLSWAGAFDY | - | 48 |
| | GLYSNPNEPFDY | D1→G, A2→L, G3→Y, L4-S, S5→N, W6-P, A7→N, G8→E, A9→P | 54 |
| | DLDAFWRPSFDY | A2→L, G3-D, L4-A, S5→F, A7→R, G8→P, A9→S | 66 |
| VL CDR1 | SGSSSNIGSNYVS | - | 61 |
| | SGSSSNIGSNYVT | S13→T | 49 |
| | TGSSSNIGSNYVS | S1→T | 55 |
| | TGSSSNIGNNNVN | S1→T, S9→N, Y11→N, S13--7N | 67 |
| | TGSSSNIGNNSVT | S1→T, S9→N, Y11→S, S13→T | 73 |
| | SGSSSNIGSNNVT | Y11→N, S13→T | 79 |
| | SDSSSNIGSNTVS | G2→D, Y11→T | 85 |
| | SGSSSNIGNNDVY | S9→N, Y11→D, S13→Y | 91 |
| VL CDR2 | SDSHRPS | - | 62 |
| | SDSHRPS | - | 50 |
| | DDSQRPS | S1→D, H4→Q | 56 |
| | SDSHRPS | - | 68 |
| | ADNNRPS | S1→A, S3→N, H4→N | 74 |
| | ANSNRPS | S1→A, D2→N, H4→N | 80 |
| | ADNNRPS | S1→A, S3→N, H4→N | 86 |
| | SDSQRPS | H4→Q | 92 |
| VL CDR3 | ATWDSSLNGYV | - | 63 |
| | GSWDYSLSAYV | A1→G, T2-S, S5→Y, N8→S, G9→A | 51 |
| | GTWDYSLNGYV | A1→G, S5→Y | 57 |
| | GSWDDSLSAYV | A1→G, T2-S, S5→D, N8→S, G9→A | 69 |
| | AAWDSSLSAYV | T2→A, N8→S, G9→A | 75 |
| | GAWDYSLSAYV | A1→G, T2→A, S5→Y, N8→S, G9→A | 81 |
| | GTWDYSLSGYV | A1→G, S5→Y, N8→S | 87 |
| | GTWDYSLSGYV | A1→G, S5→Y, N8→S | 93 |

### [Example 7] Regulation of Signaling Pathways in Regulatory T cells by Antibodies According to the Present Invention

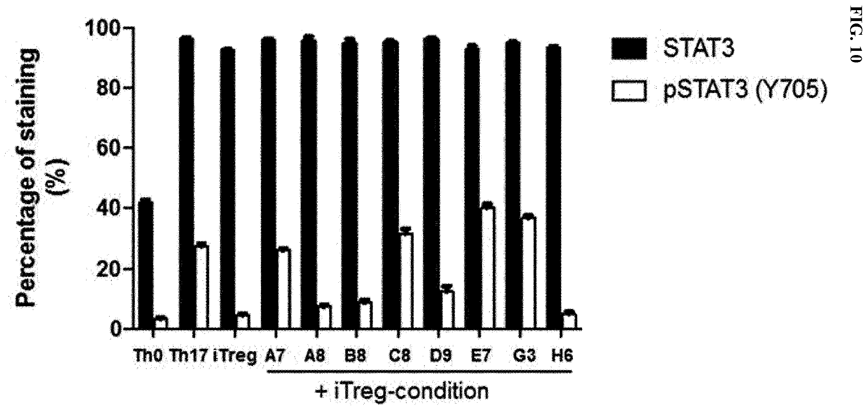

In order to analyze how the monoclonal antibodies of Preparation Examples 1 to 9 according to the present invention affect the signaling pathways in regulatory T cells through the Lrig-1 protein, regulatory T cells were treated with monoclonal antibodies corresponding to Preparation Examples 1 to 9 to stimulate Lrig-1 present on the surface of the regulatory T cells. Then, the level of tyrosine phosphorylation of Stat3 protein present in the stimulated regulatory T cells was analyzed by phosphotyrosine immunoblotting. The results are shown in FIG. 10.

As shown in FIG. 10, it could be found that the Lrig-1 protein-specific monoclonal antibodies (GTC210-01, GTC210-02, GTC210-03 and GTC210-04) according to the present invention increased phosphorylation of Stat3 to the same level as that in Th17 cells. Meanwhile, it could be found that the Lrig-1 protein-specific monoclonal antibodies (GTC110-01, GTC110-02, GTC110-03 and GTC110-04) according to the present invention continued to maintain and decrease the phosphorylation of Stat3 at the same level as that in iTreg cells.

### [Example 81 Evaluation of Therapeutic Effects of Preparation Examples 1 to 4

### [8-1] Evaluation of Therapeutic Effect on Immune-Related Disease

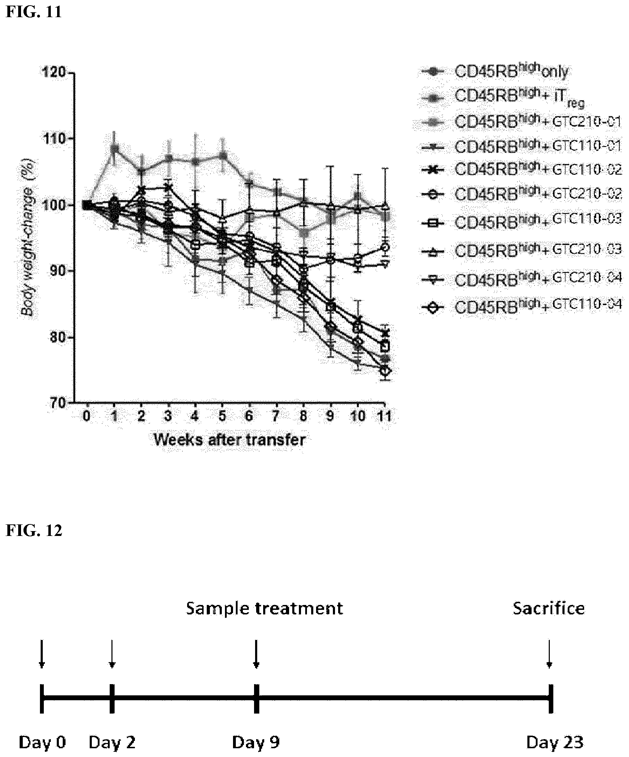

In order to evaluate the therapeutic effects of the monoclonal antibodies of Preparation Examples 1 to 4 according to the present invention on autoimmune disease, RAG-1^{-/-} mice were subjected to adoptive transfer with CD45RB (high) cells to induce inflammatory bowel disease (IBD), which is autoimmune disease. Then, the antibodies of Preparation Examples 1 to 4 were intraperitoneally injected in an amount of 200 µg/mouse, and then therapeutic effects thereof on the autoimmune disease were analyzed. The results are illustrated in FIG. 11.

As shown in FIG. 11, it could be found that the Lrig-1 protein-specific monoclonal antibodies (GTC210-01, GTC210-02, GTC210-03 and GTC210-04) according to the present invention remarkably inhibited the weight loss effect in the inflammatory bowel disease-induced mice.

Thereby, it can be seen that the Lrig-1 protein-specific monoclonal antibody according to the present invention are capable of effectively preventing, ameliorating, or treating immune-related diseases, such as autoimmune disease, graft-versus-host disease, organ transplant rejection, asthma, atopy, or acute or chronic inflammatory disease, which are induced by excessive activation and expression of various immune cells and inflammatory cells.

### [8-2] Evaluation of Therapeutic Effect on Multiple Sclerosis

### 1. Evaluation of Therapeutic Effect of GTC210-01 Antibody on Multiple Sclerosis

To produce multiple sclerosis mouse models, an experiment was conducted according to the experimental design shown in FIG. 12. On day 0, MOG peptide was injected subcutaneously into 7-week-old C57BL/6 mice, and Mycobacterium tuberculosis toxin was injected intraperitoneally. On day 2, Mycobacterium tuberculosis toxin was injected once more to strengthen the immune system of the mice. On day 9, the GTC210-01 antibody produced in Preparation Example 1 was administered to the mice, and an anti-IL-17a antibody was administered as a positive control.

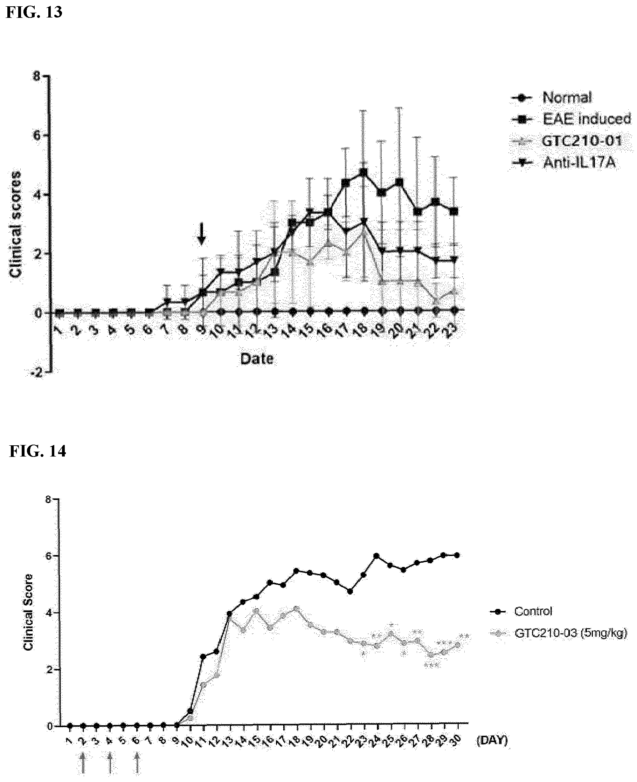

Thereafter, starting from day 7, the therapeutic effect on multiple sclerosis in the mice was evaluated by calculating the clinical scores according to the following evaluation criteria, and the results are shown in FIG. 13.

### <Evaluation Criteria>

0 points: Normal movement with no symptoms.
1 point: Part of the tip of the tail is paralyzed and hangs down.
2 points: The entire tail is paralyzed and is limp.
3 points: One of the hindlimbs is partially paralyzed but is able to respond to stimuli.
4 points: One of the hindlimbs is completely paralyzed and does not respond to stimuli, causing the limb to drag when moving.
5 points: Both hindlimbs are paralyzed and body dragging is made with the forelimbs.
6 points: It is difficult to make body dragging with the forelimbs and the forelimbs still respond to stimuli.
7 points: The mouse is unable to move but one forelimb responds to stimuli.
8 points: The mouse is unable to move and both forelimbs do not respond to stimuli.
9 points: The mouse is unable to move and has irregular breathing.
10 points: The mouse dies.

As shown in FIG. 13, it could be found that, when the antibody according to the present invention was administered to the model with induced multiple sclerosis, the multiple sclerosis was remarkably treated. In particular, it could be found that, on days 22 and 23, the model was recovered to a level similar to that of normal mice.

### 2. Evaluation of Therapeutic Effect of GTC210-03 Antibody on Multiple Sclerosis

To produce multiple sclerosis mouse models, an experiment was conducted according to the experimental design shown in FIG. 12. On day 0, MOG peptide was injected subcutaneously into 7-week-old C57BL/6 mice, and Mycobacterium tuberculosis toxin was injected intraperitoneally. On day 2, Mycobacterium tuberculosis toxin was injected once more to strengthen the immune system of the mice. Next, on days 2, 4 and 6, the GTC210-03 antibody produced in Preparation Example 3 was administered to the mice at a concentration of 5 mg/kg.

Thereafter, starting from day 10, the therapeutic effect on multiple sclerosis in the mice was evaluated by calculating the clinical scores according to the following evaluation criteria, and the results are shown in FIG. 14.

### <Evaluation Criteria>

0 points: Normal movement with no symptoms.
1 point: Part of the tip of the tail is paralyzed and hangs down.
2 points: The entire tail is paralyzed and is limp.
3 points: One of the hindlimbs is partially paralyzed but is able to respond to stimuli.
4 points: One of the hindlimbs is completely paralyzed and does not respond to stimuli, causing the limb to drag when moving.
5 points: Both hindlimbs are paralyzed and body dragging is made with the forelimbs.
6 points: It is difficult to make body dragging with the forelimbs and the forelimbs still respond to stimuli.
7 points: The mouse is unable to move but one forelimb responds to stimuli.
8 points: The mouse is unable to move and both forelimbs do not respond to stimuli.
9 points: The mouse is unable to move and has irregular breathing.
10 points: The mouse dies.

As shown in FIG. 14, it could be found that, when the antibody according to the present invention was administered to the model with induced multiple sclerosis, the multiple sclerosis was remarkably treated. In particular, it could be found that, unlike the positive control group in which the condition gradually worsened, the condition in the mice to which the GTC210-03 antibody of Preparation Example 3 was administered began to improve starting from about 18 days.

### 3. Identification of Reduction in Demyelination by GTC210-03 Antibody

In order to identify whether demyelination was reduced when the GTC210-03 antibody of Preparation Example 3 was administered, verification was made through immunohistochemical analysis. On day 21st day after disease induction, when the mice were in the worst condition, the spinal cords were obtained from the mice, and the degree of myelin destruction and the degree of infiltration of inflammatory cells were checked.

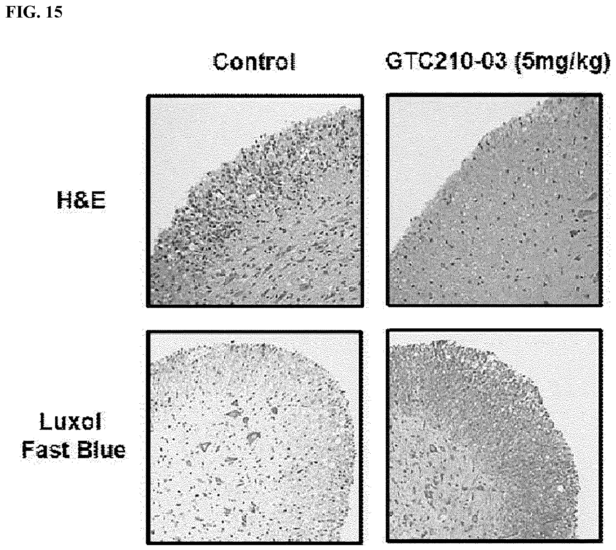

First, for H&E staining, the lungs and bronchial tubes of the animal model from which red blood cells have been removed by washing with PBS were stored in formaldehyde for 24 hours, and then paraffin tissue sections were prepared. Next, a dye was added to xylene for deparaffinization, and then the xylene was removed using 100% alcohol. Next, the mouse nervous tissue was subjected to hematoxylin staining, followed by differential decolorization (differentiator). Then, blueing was performed, followed by counter-staining with eosin. In addition, Luxol Fast Blue dye was used to check the degree of myelin destruction. This dye is a dye for staining the myelin sheath, and if the disease has advanced significantly, the dye does not work properly. Specifically, Luxol Fast Blue was also added to xylene for deparaffinization, and then the xylene was removed using 100% alcohol. Next, the mouse nervous tissue was subjected to Luxol Fast Blue staining at 60°C for 16 to 24 hours, followed by differential decolorization (differentiator). The results are shown in FIG. 15.

As shown in FIG. 15, as a result of each of H&E staining and Luxol fast blue staining, it could be found that, the GTC210-03 antibody of Preparation Example 3 was administered, demyelination was noticeably reduced compared to that in the control group.

### 4. Identification of Reduced Inflammatory Response in Mouse Disease Model of Experimental Autoimmune Encephalomyelitis (EAE)

A mouse disease model of experimental autoimmune encephalomyelitis (hereinafter referred to as EAE), which is a representative animal model, was used to identify reduced inflammatory response in immune-related disease, that is, autoimmune disease. Splenocytes were isolated from the mice treated with the GTC210-03 antibody of Preparation Example 3, and CD4 T cells were reactivated. In this case, since the CD4 T cells have already been exposed to the antigen *in vivo* in the mice, when they are stimulated *ex vivo*, stronger activation occurs and the differentiated CD4 T cells secrete their representative cytokines. In the case of the mice, cells were obtained on day 10 after disease induction and reactivated with 40 µg/ml of MOG peptide for 48 hours. Thereafter, the amount of cytokines present in the culture medium was analyzed through ELISA.

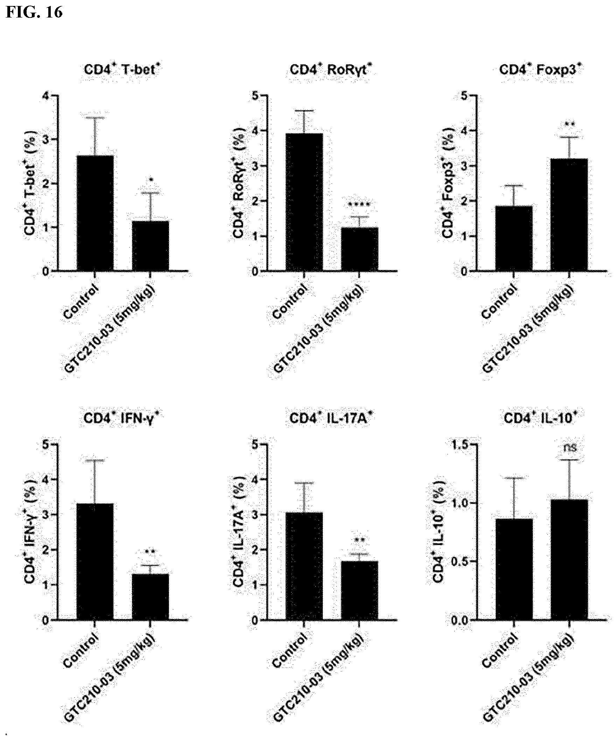

As a result, as shown in FIG. 16, it could be found that, when the GTC210-03 antibody of Preparation Example 3 was administered, expression of helper T cell 1 (CD4+ T-bet+) and helper T cell 17 (CD4+ RoRγt+) decreased, expression of regulatory T cells (CD4+ Foxp3+) increased, expression of various inflammatory cytokines (IFN-gamma and IL-17A) decreased, and expression of anti-inflammatory cytokine (IL-10) increased. Therefore, it could be found that, when the GTC210-03 antibody of Preparation Example 3, which binds specifically to the epitope according to the present invention, was administered, it not only inhibited the expression of "inflammatory" cytokines, but also increased the expression of the "anti-inflammatory" cytokine IL-10, thereby suppressing the inflammatory response.

### [8-3] Evaluation of Therapeutic Effect Against Alzheimer's Disease

In order to evaluate the therapeutic ability of the antibody according to the present invention against Alzheimer's disease, groups were designed as shown in Table 6 below. Specifically, 7-month-old 5xFAD mice with induced Alzheimer's disease (known to exhibit amyloid deposition, gliosis, and progressive neuronal loss with cognitive and motor deficits) were intravenously injected with each of GTC110-04 mouse antibody and GTC210-01 antibody in an amount of 10 mpk for 1 month. As a positive control, glatiramer acetate (GA, Copaxone^{®}) was injected subcutaneously into 8-month-old mice with Alzheimer's disease in an amount of 100 µg for 3 weeks.

**[Table 6]**

| Group | | | n/group | Administration route | Dose |
|---|---|---|---|---|---|
| G1 | WT | Control | 14 | - | - |
| G2 | 5xFAD | Control | 13 | - | - |
| G3 | | GTC 110-04 antibody | 11 | IV | 10 mpk |
| G4 | | GTC210-01 antibody | 11 | IV | 10 mpk |
| G5 | | GA (positive control) | 11 | SC | 100µ g |

Then, a Y-maze test, a novel object recognition test, and a water maze test were performed on the mice of the above groups.

### 1. Y-Maze Test

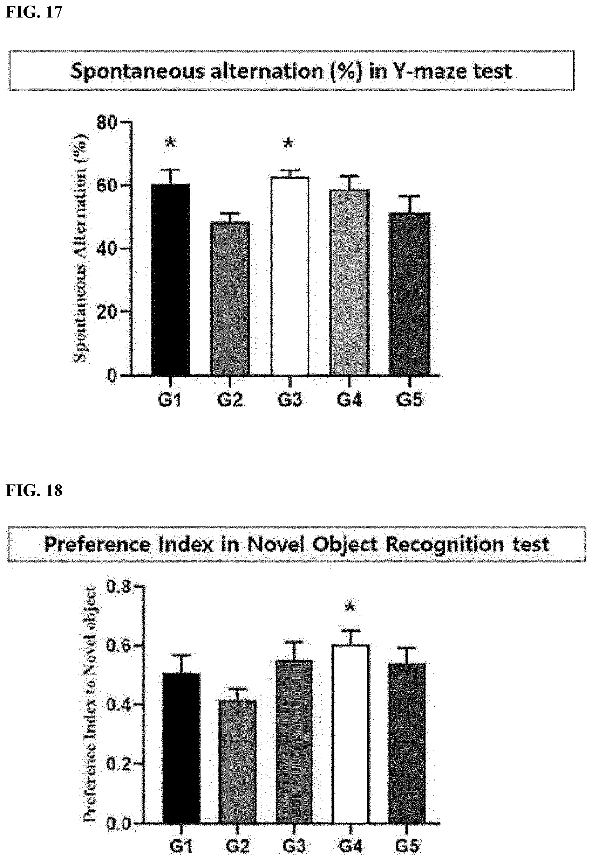

Specifically, for the Y-maze test, a Y-shaped maze was used which had three identical arms having a length of 40 cm (with a 15-cm high wall) at an angle of 120 degrees from each other. This experiment was a behavioral experiment using the rodent's instinctive exploring habit and was based on the fact that there is a high possibility of exploring a new area. The higher the degree that the test animal remembered the arm it had just explored and tried not to enter the same arm, the higher the animal's memory level. An exploring time of 8 minutes was given to each individual, and the final results were expressed as spontaneous alteration (%) values in FIG. 17. The spontaneous alteration (%) value was calculated by Expression 1 below. Here, behavioral patterns were analyzed using SMART VIDEO TRACKING Software (Panlab, USA). The results are shown in FIG. 17.

### [Expression 1]

### Spontaneous alternation (%) = the number of triplet / (total arm entry-2)

As shown in FIG. 17, in this experiment for measuring the relative frequency at which the test animal perceives the surrounding clues and sequentially enters the maze, it could be found that, when the antibodies (GTC210-01 and GTC110-04 antibodies) according to the present invention were administered to the mice with induced Alzheimer's disease, the mice had a spontaneous alternation value at a level similar to that of a normal control.

### 2. Novel Object Recognition Test

The novel object recognition test was performed to evaluate memory using two different objects in a 40 cm×40 cm acrylic cage. After causing the test animal to acclimatize to the inside of the acrylic cage, two objects were placed at certain locations and the animal was allowed to perceive the objects freely. Then, the time for exploring each object was measured. 24-hour delay was given to each individual, and only one object was changed to another object at the same location. Here, in a case where the animal perceives the changed object as a novel object and spends a longer exploring time, it may be determined that the animal has better memory. In a case where the animal does not remember the objects that it explored 24 hours before, the animal fails to distinguish between the novel object and the old object, and thus there is a possibility that the animal explores equally both objects. The animal was allowed to explore freely for a total of 10 minutes, and the result was expressed as a preference index (which equals novel object exploring time/total exploring time) in FIG. 18. Here, the analysis was performed using SMART VIDEO TRACKING Software (Panlab, USA).

As shown in FIG. 18, regarding the preference index for the novel object, the normal control (G1) was measured to be 0.51±0.06, and the G2 group (vehicle) was measured to be 0.42 ± 0.04. Thus, it was observed that the group with induced Alzheimer's disease (G2) had a low preference index as compared with the normal control (G1). On the other hand, it could be found that the group having received the antibodies (GTC210-01 and GTC110-04 antibodies) according to the present invention had a higher preference index than the normal control.

### 3. Water Maze Test

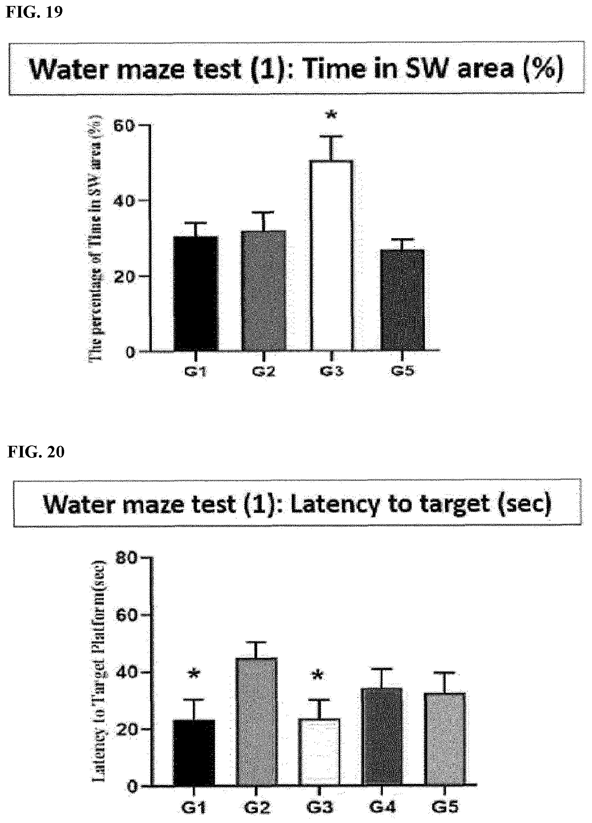

The water maze test was conducted based on the method devised by Morris. A stainless steel pool (90 cm in diameter, 50 cm in height) was filled with water (22±1°C) so that the height of the water surface was 30 cm. A hidden platform (5 cm in diameter) was placed 1 cm below the water surface. On day 1 of evaluation, 3 to 4 training sessions were allowed. A total swimming time per individual was set to 60 seconds; and the individual, that found the platform within 60 seconds, was allowed to stay on the platform for 10 seconds to induce memory. The individual, that was unable to find the platform even after 60 seconds, was manually guided to the platform and allowed to stay on the platform for 10 seconds, wherein the time to escape was set to 60 seconds. On day 6 after the swimming training, a probe test was conducted to measure the value (percentage of time in SW area, %), which is spatial perception, obtained by calculating the time spent in the quadrant where the platform was as a percentage of the total swimming time, and the time (latency to target, sec) taken to reach the location where the platform was. The results are shown in FIGS. 19 and 20. Here, the analysis was performed using SMART VIDEO TRACKING Software (Panlab, USA). In interpreting the results, the individual included in the exclusion criteria was set as an individual that moved around a specific area without exploration through swimming.

As shown in FIGS. 19 and 20, it could be found that the group having received the antibodies (GTC210-01 and GTC110-04 antibodies) according to the present invention had a remarkably increased value (percentage of time in SW area, %), obtained by calculating the time spent in the quadrant where the platform was as a percentage of the total swimming time, and had a remarkably decreased time (latency to target, sec) taken to reach the location where the platform was, which was a level similar to that of the normal control.

### [Example 91 Identification of Epitope of Lrig-1 Protein

The following experiment was performed to identify a structural epitope, is a site to which the GTC210-03 antibody of Preparation Example 3 can bind, in the extracellular domain of the Lrig-1 protein.

### [9-1] Reagents and Materials

CNBr-activated Sepharose^{™} 4B) (GE, 17-0430-01)
Diethylpyrocarbonate (DEPC, Sigma, 159220)
Imidazole (Acros, 301870010)
Sequencing grade modified trypsin (Promega, V5117)
Chymotrypsin, sequencing grade (Promega, V1062)
Asp-N, sequencing grade (Promega, V1621)
rLyc-C, mass spec grade (Promega, V1671)
Disuccinimidyl dibutyric urea (DSBU; Thermo, A35459)
Dimethyl sulfoxide (DMSO, Sigma, D5879)
Sodium bicarbonate (NaHCOs, Sigma, 792519)
Sodium chloride (NaCl, Sigma, S3014)
Sodium acetate (NaOAc, Sigma, S2889)
Tris (GE, 17-1321-01), glycine) (GE, 17-1323-01)
ProteaseMAX^{™} surfactant, trypsin enhancer (Promega, V207A)
Acetone (Merck, 1.07021.2521)
Dithiothreitol (DTT; GE, 17-1318-02)
Iodoacetamide (IAA; Sigma, I-6125)
Sodium phosphate monobasic dihydrate (NaH₂PO₄, Sigma, 71505)
Sodium phosphate dibasic hepta-hydrate (Na₂HPO₄, Sigma, S9390)
Syringe filter 0.2-µm (Sartorius Stedim, 16534)
Acetonitrile, methanol, water (HPLC grade, J.T baker)
Formic acid (Sigma, 33015)

### [9-2] Experimental Methods

### [9-2-1] Covalent Labelling MS (CL-MS)

The ratio of antigen and antibody was set to 2:1, and an antigen sample was prepared. Then, DEPC was added thereto so as not to exceed 1%, and then reaction was performed at 37°C for 1 minute and terminated by adding imidazole. Next, a precipitation reaction was performed by adding acetone, and then the supernatant was removed by centrifugation, and the precipitate was well dissolved using 0.1% PM. The dissolved precipitate was degraded by adding trypsin, chymotrypsin, or Asp-N/Lys-C thereto, and then the sugar chains attached to the protein were removed using PNGase-F. Finally, disulfide bonds existing between cysteines of the protein were cleaved using DTT and IAA, and LC-MS and MS2 analysis were performed.

### [9-2-2] Cross-Linking MS (XL-MS)

The ratio of antigen and antibody was set to 2:1, and two antigen samples were prepared. Then, a cross-linker (CSBU) was sufficiently dissolved by DMSO. Thereafter, the sample and DSG were added at a ratio of 1:100, and only DMSO was added to the other sample, and then reaction was performed at 25°C for 1 hour. After the reaction was completed, the protein was degraded using trypsin or chymotrypsin, and then the sugar chains attached to the protein were removed using PNGase-F. Finally, disulfide bonds existing between cysteines of the protein were cleaved using DTT and IAA, and LC-MS and MS2 analysis were performed.

### [9-2-3] LC-MS and MS Analysis Conditions

### 1. Liquid chromatography (LC) conditions

Column: Hypersil GOLD^{™} (1.9 µm, 1 × 100 mm)
Flow rate: 100 µL/min
Mobile phase A: D.W. / 0.2% formic acid
Mobile phase B: ACN / 0.2% formic acid
Temperature): 30°C
LC condition (gradient)

**[Table 7]**

| Step | Total time (min) | Flow rate (µL/min) | A(%) | B(%) |
|---|---|---|---|---|
| 0 | 0.00 | 100.00 | 95.0 | 5.0 |
| 1 | 5.00 | 100.00 | 95.0 | 5.0 |
| 8 | 7.00 | 100.00 | 90.0 | 10.0 |
| 3 | 48.00 | 100.00 | 70.0 | 30.0 |
| 4 | 55.00 | 100.00 | 5.0 | 95.0 |
| 5 | 65.00 | 100.00 | 5.0 | 95.0 |
| 6 | 66.00 | 100.00 | 95.00 | 5.0 |
| 7 | 71.00 | 100.00 | 95.00 | 5.0 |

### 2. MS and MS2 conditions

**[Table 8]**

| HESI source | |
|---|---|
| Sheath gas flow rate | 35 |
| Auxiliary gas flow rate | 10 |
| Sweep gas flow rate | 1 |
| Spray voltage (kV) | 3.5 |
| Spray current (µA) | - |
| Capillary temperature (°C) | 250 |
| S-lens RF level | 50 |
| Auxiliary gas heater temperature (°C) | 200 |

**[Table 9]**

| General | |
|---|---|
| Runtime | From 0 to 71 min |
| Polarity | Positive |
| Default charge state | 2 |

**[Table 10]**

| Full MS | |
|---|---|
| Resolution | 70,000 |
| AGC target | 3.00E+06 |
| Maximum IT | 100 ms |
| Scan range | 200 to 2,000 m/z |

**[Table 11]**

| dd-MS² / dd-SIM | |
|---|---|
| Resolution | 17,500 |
| AGC target | 1.00E+05 |
| Maximum IT | 54 ms |
| Loop count | 10 |
| TopN | 10 |
| Isolation window | 1.6 m/z |
| (N)CE / stepped (N)CE | nce: 27 |

**[Table 12]**

| dd settings | |
|---|---|
| Minimum AGC target | 8.00E+04 |
| Intensity threshold | 1.50E+05 |
| Charge exclusion | Unassigned, 7, 8, >8 |
| Peptide match | Preferred |
| Exclude isotopes | On |
| Dynamic exclusion | 5.0 s |

### [9-3] Covalent labeling MS (CL-MS)

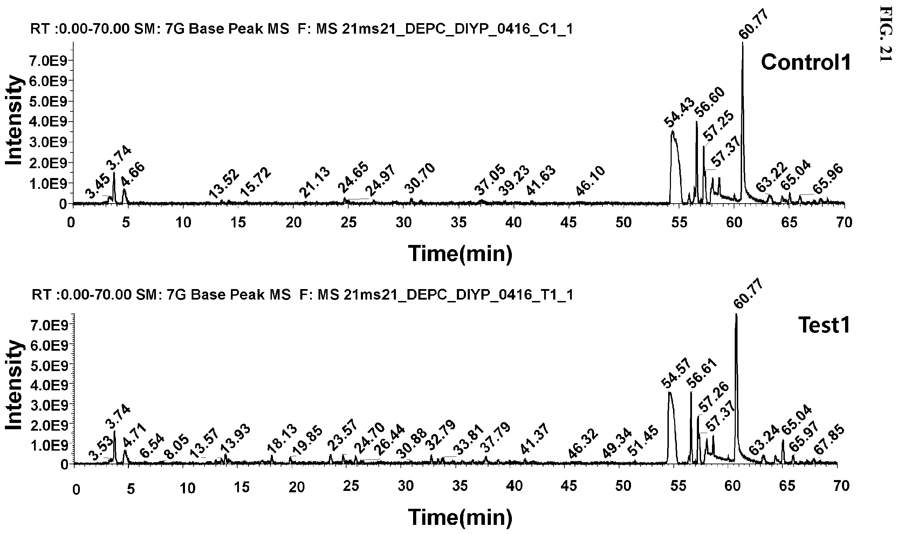

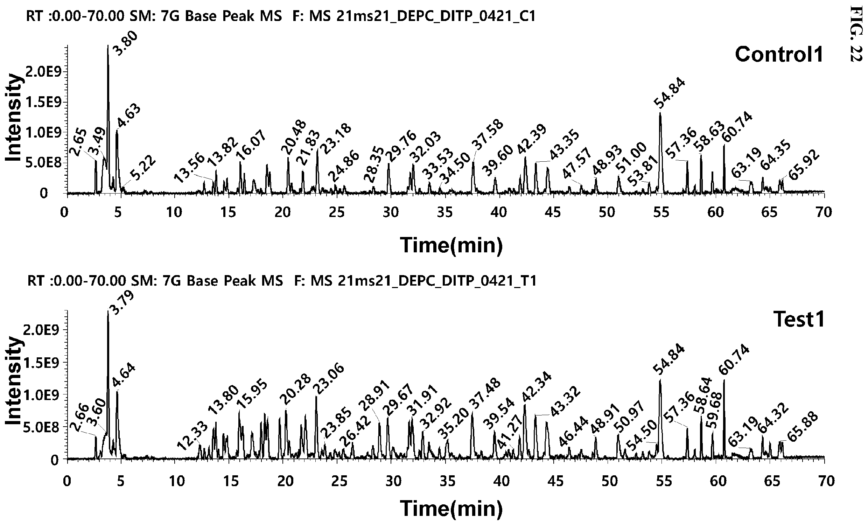

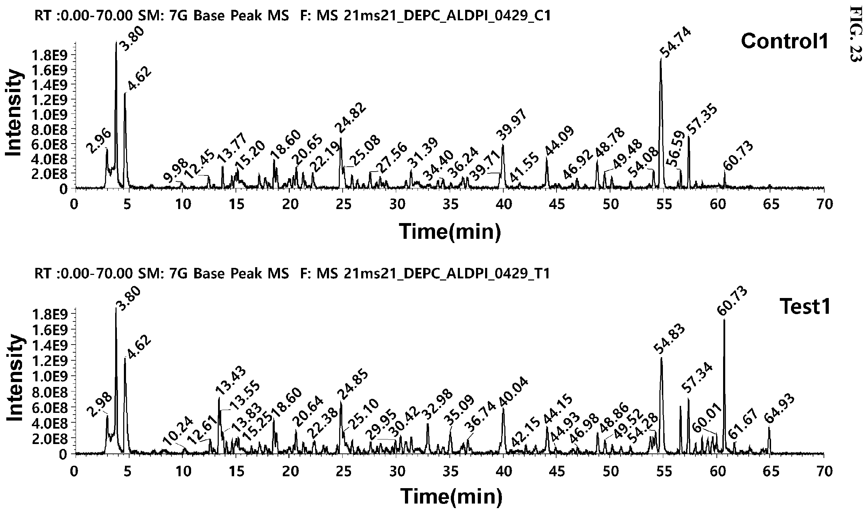

The samples, obtained by treating each of the huTregL1-his (Ag, Control) control sample and the huTregL1-his/E7-mlgG2a (Ag+Ab, Test) test sample with DEPC and then degrading the same by treatment with trypsin, chymotrypsin or Asp-N/Lys-C, were analyzed in triplicate (see FIGS. 21 to 23). The results of MS analysis of the control (Control 1) and the test (Test 1) samples, shown in the analysis results, were matched with the sequence of the control (huTregL1-his) using the BioPhama finder program, and labeled peptides were compared. In addition, the present inventors performed the process of identifying unlabeled and labeled peptides in the control and test samples treated with chymotrypsin, trypsin, or Asp-N/Lys-C. The results of the repeated tests were taken together, and the results obtained by comparing the labeling (%) of the control and test samples (decrease ≥ 5%) were summarized based on the amino acid sequence (Res#) and are shown in FIGS. 21 and 22. Based on the results thus summarized, the sequences were substituted into the huTregL1-his sequence, and then a matching sequence in the results of the two protease tests was found.

As a result, it was found that, among the sequences with a labeling decrease (%) of 5% or more, the sequences E62-L101, D111-L134, and Q542-Y553 had labeling decreases (%) of 13.2%, 15.3%, and 14.7%, respectively, which are more than 10%. In the E62-L101 sequence, the E62-K92 portion (AL) showed a labeling (%) of 65.9%, the L65-F87 portion (Y) showed a labeling (%) of 48.7%, and the L88-L101 portion showed a labeling (%) of 88.6%. These results suggest that, in the E62-L101 sequence, the L88-L101 portion with a labeling (%) of 50% or more is exposed to the outside. It was found that the D111-L134 sequence showed a labeling decrease (%) of 15.3% in the chymotrypsin-treated group, but the RSD of the control was 62.4%. The Q542-Y553 sequence also showed a labeling decrease (%) of 14.7% in the chymotrypsin-treated group, and the labeling (%) of the control antigen was 50% or more, indicating that it is an externally exposed portion of the protein structure.

It was finally determined that the present sequences derived from the CL-MS analysis results for the test sample (huTregL1-his/E7-mlgG2a) showed a labeling decrease (%) of 5% or more in two or more protease-treated groups, and that the E62-L101 sequence, which is a sequence determined to be exposed to the outside, corresponds to an epitope site.

### [9-4] Cross-linking MS (XL-MS)

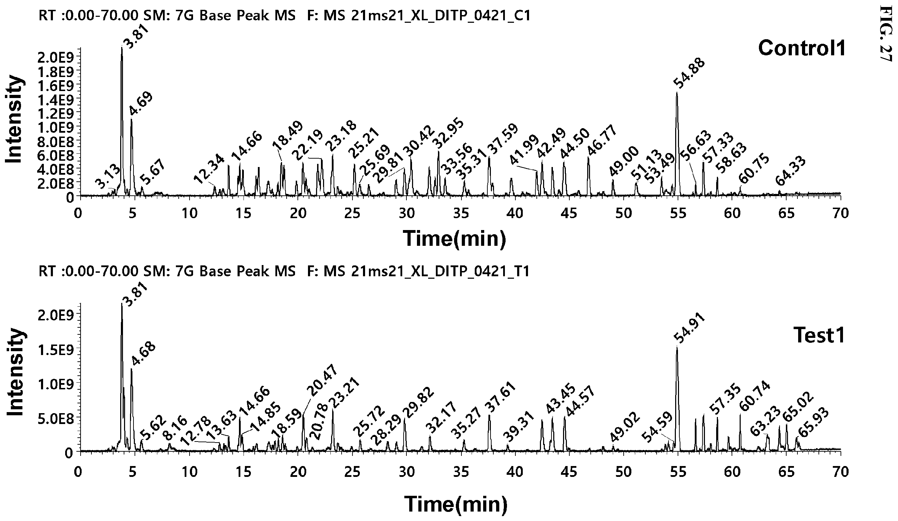

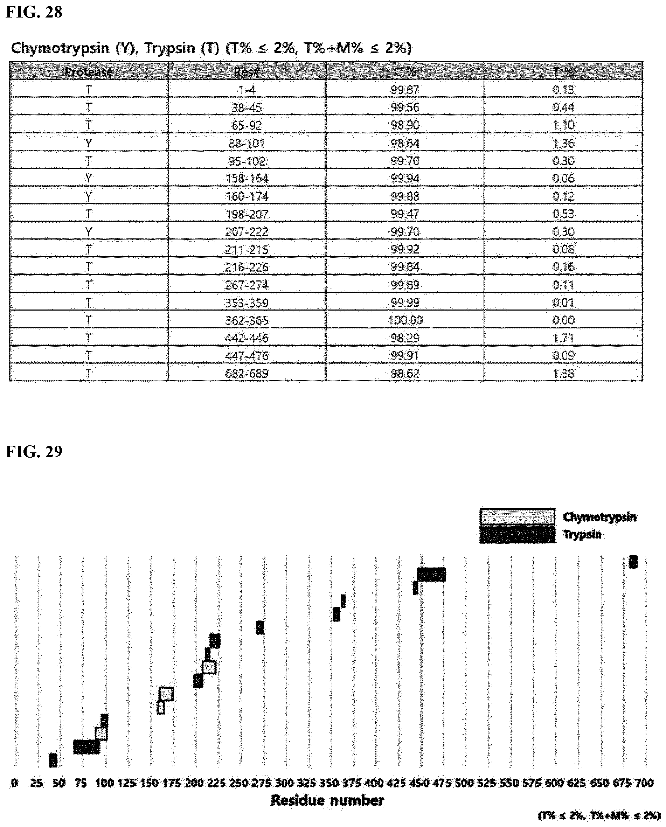

A test (huTregL1-his/E7-mlgG2a) mixture sample was prepared, treated with disuccinimidyl dibutyric urea (DSBU), a cross-linker, and degraded with protease (chymotrypsin or trypsin) (FIGS. 27 and 28). Thereafter, peptides obtained by degradation with chymotrypsin and trypsin were compared with the control, and peptide sequences that were not detected (including 2% or less) in the test tube were identified. In addition, in these peptides, the form (mono) to which only the cross-linker bound was examined and reconfirmed (M%), and finally, the peptide sequences not detected in the test samples were selected and determined to be epitope sites. For three sequences, including L88-K92, S90-L101, and N211-W222, the percentage (T%) of peptides of the same sequence detected in the test sample compared to the control group was found to be 2% or less in either chymotrypsin or trypsin. T158-F174 and G442-K476 were reduced in chymotrypsin (Y) and trypsin (T), respectively. The only sequence in which T% was not detected (0%) was S362-K365 in the trypsin (T)-treated group. Through cross-linking MS analysis, three sequences, L88-K92, S90-L101, and N211-W222, which were significantly reduced in the two protease-treated groups, were determined to be epitope sites.

### [9-5] Conclusion

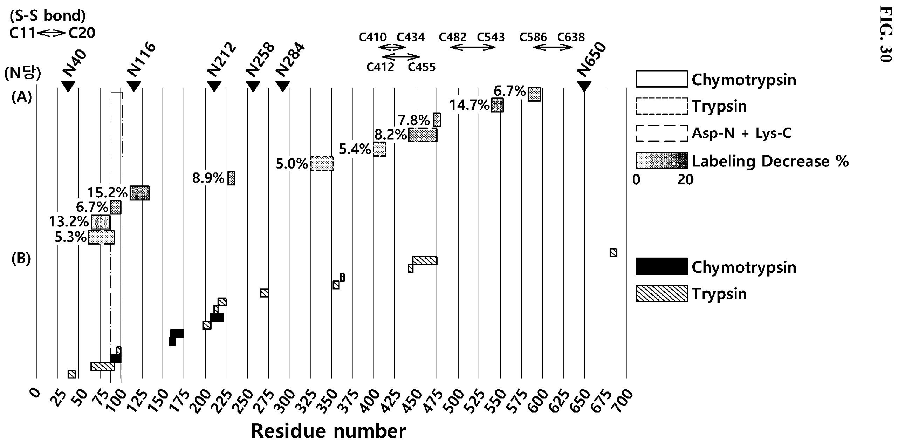

As shown in FIGS. 29 and 30, the epitope was predicted by taking the above results together. Specifically, two sequences, including E62-L101 and G452-K476 of huTregL1-his (Ag), were identified to be matching sequences in CL-MS and XL-MS analysis results. The L88-K92 portion in L62-L101 showed a labeling decrease (%) in the two protease-treated groups (chymotrypsin, and Asp-N/Lys-C) as a result of CL-MS, and was also significantly reduced in the two protease-treated groups (chymotrypsin, and trypsin) as a result of XL-MS analysis. The S95-L101 portion was an epitope identified in both chymotrypsin and trypsin in XL-MS. In addition, the results of CL-MS showed that the L88-L101 portion with a labeling (%) of 50% or more was exposed to the outside. In particular, the amino acid sequence of L88-L101 was composed of consecutive hydrophilic amino acids (Q89-S95).

In addition, in the case of G452-K476, the A472-F480 portion was found to be exposed to the outside as a result of CL-MS analysis, and in particular, the A472-K476 portion was determined to correspond to an epitope. Although N211-W222 was selected as an epitope site in XL-MS, it did not match with that in the CL-MS analysis, and furthermore, this sequence portion was had a labeling (%) of 50% or less, indicating that it was located internally in the structure. Thus, it was excluded from the epitope.

Although the present invention has been described in detail above, the scope of the present invention is not limited thereto. It will be obvious to those skilled in the art that various modifications and changes can be made without departing from the technical spirit of the present invention as described in the claims.

### Industrial Applicability

The antibody or antigen-binding fragment that binds specifically to the epitope of Lrig-1 protein according to the present invention is capable of binding specifically to the epitope of the present invention, which is present on regulatory T cells, thereby suppressing the function of the regulatory T cells and maintaining or increasing the activity of effector T cells. Thus, the antibody or the antigen-binding fragment may be very efficiently used to prevent, ameliorate or treat various diseases.

## Claims

1. An epitope of Lrig-1 (leucine-rich and immunoglobulin-like domains 1) protein, which is selected from the group consisting of polypeptides consisting of the amino acid sequences represented by SEQ ID NOs: 38, 39, 41, 42, 44 and 45.

2. The epitope according to claim 1, wherein the polypeptides consist of the amino acid sequences represented by SEQ ID NOs: 35, 36, 37, 40 and 43.

3. A nucleic acid molecule encoding the epitope according to claim 1.

4. An expression vector into which the nucleic acid molecule according to claim 3 has been inserted.

5. A host cell line transfected with the expression vector according to claim 4.

6. A binding molecule which binds specifically to an epitope comprising at least one polypeptide selected from the group consisting of polypeptides consisting of the amino acid sequences represented by SEQ ID NO: 35 to SEQ ID NO: 45.

7. The binding molecule according to claim 6, which is an antibody or a fragment thereof.

8. The binding molecule according to claim 7, wherein the antibody is a chimeric antibody, a humanized antibody, a bivalent-bispecific molecule, a minibody, a domain antibody, a bispecific antibody, an antibody mimic, a diabody, a triabody, a tetrabody, or a fragment thereof.

9. A chimeric antigen receptor (CAR) comprising an antigen-specific binding domain, a linking domain, and a CD3 zeta (ζ) signaling domain,
wherein the antigen-specific binding domain is a fusion protein comprising: an antigen-specific binding domain that binds specifically to an epitope comprising at least any one polypeptide selected from the group consisting of polypeptides consisting of the amino acid sequences represented by SEQ ID NO: 35 to SEQ ID NO: 45; and an immunoglobulin Fc region.

10. An antibody-drug conjugate comprising: the binding molecule according to claim 6; and a drug.

11. A pharmaceutical composition for preventing or treating immune-related disease comprising, as an active ingredient, any one of the binding molecule according to any one of claims 6 to 8, the chimeric antigen receptor according to claim 9, and the antibody-drug conjugate according to claim 10.

12. The pharmaceutical composition according to claim 11, wherein the immune-related disease is at least one selected from the group consisting of autoimmune disease, graft-versus-host disease, organ transplant rejection, asthma, atopy, and acute or chronic inflammatory disease.

13. The pharmaceutical composition according to claim 12, wherein the autoimmune disease is at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

14. A pharmaceutical composition for preventing or treating brain nervous system disease comprising, as an active ingredient, any one of the binding molecule according to any one of claims 6 to 8, the chimeric antigen receptor according to claim 9, and the antibody-drug conjugate according to claim 10.

15. The pharmaceutical composition according to claim 14, wherein the brain nervous system disease is neurodegenerative disease or neuroinflammatory disease.

16. The pharmaceutical composition according to claim 14, wherein the neurodegenerative disease or neuroinflammatory disease is at least one selected from the group consisting of stroke, dementia, Alzheimer's disease, Parkinson's disease, Huntington's disease, Niemann-Pick disease, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.

17. A method of screening a binding molecule, which is an antibody or a fragment thereof, using the epitope according to claim 1 or 2.

18. A method of screening an epitope of Lrig-1 protein using the antibody according to claim 6.

19. A method of binding a binding molecule, which is an antibody or a fragment thereof, to the epitope according to claim 1 or 2.

20. A binding molecule, which is an antibody or a fragment thereof that binds specifically to Lrig-1 (leucine-rich and immunoglobulin-like domains 1) protein, the binding molecule comprising:
(a) a heavy-chain variable region (VH) comprising:
a heavy-chain variable region (VH) CDR1 selected from the group consisting of the amino acid sequences represented by SEQ ID NOs: 46, 52, 58, 64, 70, 76, 82 and 88;
a heavy-chain variable region (VH) CDR2 selected from the group consisting of the amino acid sequences represented by SEQ ID NOs: 47, 53, 59, 65, 71, 77, 83 and 89; and
a heavy-chain variable region (VH) CDR3 selected from the group consisting of the amino acid sequences represented by SEQ ID NOs: 48, 54, 60, 66, 72, 78, 84 and 90; and
(b) a light-chain variable region (VL) comprising:
a light-chain variable region (VL) CDR1 selected from the group consisting of the amino acid sequences represented by SEQ ID NOs: 49, 55, 61, 67, 73, 79, 85 and 91;
a light-chain variable region (VL) CDR2 selected from the group consisting of the amino acid sequences represented by SEQ ID NOs: 50, 56, 62, 68, 74, 80, 86 and 92; and
a light-chain variable region (VL) CDR3 selected from the group consisting of the amino acid sequences represented by SEQ ID NOs: 51, 57, 63, 69, 75, 81, 87 and 93,
wherein the binding molecule binds specifically to an epitope comprising at least any one polypeptide selected from the group consisting of polypeptides consisting of the amino acid sequences represented by SEQ ID NO: 35 to SEQ ID NO: 45.
